# EUROPÄISCHE PATENTSCHRIFT

(11) **EP 2 325 176 B1**
(45) Veröffentlichungstag und Bekanntmachung des Hinweises auf die Patenterteilung: **25.02.2015**
(21) Anmeldenummer: 10177802.5
(22) Anmeldetag: 04.05.1999
(51) Int. Cl.: C07D 261/04, C07D 413/10, C07C 251/40, C07C 319/14

(54) **Verfahren zur Herstellung von Isoxazolin-3-yl-Acylbenzolen**
Process for the preparation of Isoxazolin-3-yl-Acylbenzenes
Procede pour la preparation d' Isoxazolin-3-yl-Acylbenzenes

(30) Priorität: 11.05.1998 DE 19820722; 12.11.1998 DE 19852095
(43) Veröffentlichungstag der Anmeldung: 25.05.2011
(62) Teilanmeldung aus: 07104691.6
(73) Patentinhaber: BASF SE, 67056 Ludwigshafen (DE)
(72) Erfinder: Rheinheimer, Joachim, 67063 Ludwigshafen (DE); von Deyn, Wolfgang, 67435 Neustadt (DE); Gebhardt, Joachim, 67157 Wachenheim (DE); Rack, Michael, 69214 Eppelheim (DE); Lochtman, Rene, 68163 Mannheim (DE); Götz, Norbert, 67547 Worms (DE); Keil, Michael, 67251 Freinsheim (DE); Witschel, Matthias, 67098 Bad Dürkheim (DE); Hagen, Helmut, 67227 Frankenthal (DE); Mißlitz, Ulf, 67433 Neustadt (DE); Baumann, Ernst, 67346 Speyer (DE)

(56) Entgegenhaltungen:
- EP-A1- 0 655 446
- EP-A1- 0 805 151
- EP-A1- 1 031 573
- WO-A-96/26200
- WO-A1-98/31682
- DE-C- 107 095
- LAPWORTH, ARTHUR: "The form of change in organic compounds, and the function of the .alpha.-meta-orientating groups", JOURNAL OF THE CHEMICAL SOCIETY, TRANSACTIONS ( 1901 ), 79, 1265-1284 CODEN: JCHTA3; ISSN: 0368-1645, 1. Januar 1901 (1901-01-01), XP002629651,
- BODOR, NICOLAE ET AL: "Structure and reactivity in the substituted nitrobenzene series. I. Kinetics and mechanism of the isonitrosation of nitro(alkyl)benzenes in alkaline solution", REVUE ROUMAINE DE CHIMIE ( 1966 ), 11(3), 393-404 CODEN: RRCHAX; ISSN: 0035-3930, 1. Januar 1965 (1965-01-01), XP002629652,
- WALTER ROTTIG: "Über eine einfache Synthese des antiken Purpurs", JOURNAL FÜR PRAKTISCHE CHEMIE, Bd. 142, 1935, Seiten 35-36, XP002111133,
- DATABASE CA [Online] CHEMICAL ABSTRACTS SERVICE, COLUMBUS, OHIO, US; 1. Januar 1984 (1984-01-01), RAHMAN, LOAY K. A. ET AL: "7-Substituted benzo[b]thiophenes and 1,2-benzisothiazoles. Part 2. Chloro and nitro derivatives", XP002629653, gefunden im STN Database accession no. 1984:406966 & RAHMAN, LOAY K. A. ET AL: "7-Substituted benzo[b]thiophenes and 1,2-benzisothiazoles. Part 2. Chloro and nitro derivatives", JOURNAL OF THE CHEMICAL SOCIETY, PERKIN TRANSACTIONS 1: ORGANIC AND BIO-ORGANIC CHEMISTRY (1972-1999) ( 1984 ), (3), 385-90 CODEN: JCPRB4; ISSN: 0300-922X, 1. Januar 1972 (1972-01-01),
- DATABASE CA [Online] CHEMICAL ABSTRACTS SERVICE, COLUMBUS, OHIO, US; 1. Januar 1983 (1983-01-01), EUERBY, MELVIN R. ET AL: "Cyclization of benzylaminonitriles. Part 5. Alkylthio and arylthio activating groups", XP002629654, gefunden im STN Database accession no. 1983:52872 & EUERBY, MELVIN R. ET AL: "Cyclization of benzylaminonitriles. Part 5. Alkylthio and arylthio activating groups", JOURNAL OF CHEMICAL RESEARCH, SYNOPSES ( 1982 ), (9), 240-1 CODEN: JRPSDC; ISSN: 0308-2342, 1. Januar 1982 (1982-01-01),
- NAGATA, KOICHI: "Thiohydroxamic acids and their metal chelates. VII. Syntheses of some compounds possessing mercapto and hydroxyimino groups and their reactivities with metal ions", CHEMICAL & PHARMACEUTICAL BULLETIN ( 1969 ), 17(4), 661-8 CODEN: CPBTAL; ISSN: 0009-2363, 1. Januar 1969 (1969-01-01), XP002629655,
- DATABASE CA [Online] CHEMICAL ABSTRACTS SERVICE, COLUMBUS, OHIO, US; 1. Januar 1967 (1967-01-01), MARTANI, ALFIO ET AL: "Thionaphthene derivatives", XP002629656, gefunden im STN Database accession no. 1967:443616 & MARTANI, ALFIO ET AL: "Thionaphthene derivatives", ANNALI DI CHIMICA (ROME, ITALY) ( 1967 ), 57(2), 121-8 CODEN: ANCRAI; ISSN: 0003-4592, 1. Januar 1967 (1967-01-01),
- DATABASE CA [Online] CHEMICAL ABSTRACTS SERVICE, COLUMBUS, OHIO, US; HODGSON, H. H. ET AL: "Sulfur derivatives of substituted benzaldehydes", XP002629657, gefunden im STN Database accession no. 1928:3388 & HODGSON, H. H. ET AL: "Sulfur derivatives of substituted benzaldehydes", JOURNAL OF THE CHEMICAL SOCIETY ( 1927 ) 2423-8 CODEN: JCSOA9; ISSN: 0368-1769, 1. Januar 1927 (1927-01-01),

## Beschreibung

Gegenstand der vorliegenden Erfindung ist ein Verfahren zur Herstellung von Isoxazolin-3-yl-acylbenzolen, neue Zwischenprodukte sowie neue Verfahren zur Herstellung dieser Zwischenprodukte.

Isoxazolin-3-yl-acylbenzole sind wertvolle Verbindungen, die im Bereich des Pflanzenschutzes eingesetzt werden können. Beispielsweise werden in WO 98/31681 2-Alkyl-3-(4,5-dihydroisoxa-zol-3-yl)-acylbenzole als herbizide Wirkstoffe beschrieben.

Aufgabe der vorliegenden Erfindung war es, ein alternatives Verfahren zur Herstellung von 3-Heterocyclyl-substituierten Benzoylderivaten zur Verfügung zu stellen. Das in WO 98/31681 beschriebene Herstellungsverfahren der 2-Alkyl-3-(4,5-dihydroisoxazol-3-yl)-acylbenzole bzw. deren Vorstufe (2-Alkyl-3-(4,5-dihydroisoxazol-3-yl)-brombenzol-Derivate) ist für die großtechnische Herstellung dieser Verbindungen nicht gut geeignet, da die Synthese über mehrere Stufen erfolgt und die Ausbeute des jeweiligen Endproduktes, bezogen auf die in der ersten Synthesestufe eingesetzten Ausgangsprodukte, relativ gering ist.

Die Herstellung von Verbindungen bzw. Zwischenverbindungen, die strukturell den Verbindungen der Formel I ähnlich sind, ist aus der Literatur bekannt:
Aus der WO 96/26206 ist ein Verfahren zur Herstellung von 4-[3-(4,5-Dihydroisoxazol-3-yl)benzoyl]-5-hydroxypyrazolen bekannt, wobei in der letzten Stufe ein 5-Hydroxypyrazol mit einem 3-(4,5-Dihydroisoxazol-3-yl)-benzoesäurederivat umgesetzt wird. Das für dieses Verfahren benötigte 3-(4,5-Dihydro-isoxazol-'3-yl)-benzoesäurederivat ist nur schwer und über eine Vielzahl von Stufen zugänglich. Insofern ist das Verfahren relativ kostenaufwendig und wirtschaftlich nicht optimal.

In DE 197 09 118 wird ein Verfahren zur Herstellung von 3-(4,5-Dihydroisoxazol-3-yl)benzoesäuren, ausgehend von 3-Brom-(4,5-dihydroisoxazol-3-yl)benzol, Grignardverbindungen und Kohlendioxid beschrieben.

Überraschenderweise wurde gefunden, daß die Anzahl der Verfahrensschritte zur Herstellung der 3-Heterocyclyl-substituierten Benzoylderivate im Vergleich zu dem in WO 98/31681 beschriebenen Verfahren reduziert werden kann, wenn die Synthese über ausgewählte Zwischenverbindungen erfolgt. Außerdem hat das Verfahren den Vorteil, daß die Gesamtausbeute an Endprodukten der Formel I bzw. auch für die Zwischenprodukte X, bezogen auf die eingesetzten Ausgangsstoffe, höher ist als die Ausbeute nach den in WO 98/31681 beschriebenen Verfahren. Darüberhinaus lassen sich die jeweiligen Zwischenprodukte bei den einzelnen Verfahrensstufen in guter Ausbeute erhalten. Außerdem sind einige der einzelnen Verfahrensstufen für die technische Herstellung der Zwischenverbindungen vorteilhaft, da sie deren kostengünstige und wirtschaftliche Herstellung ermöglichen. Ferner ist vorteilhaft, daß es sich bei den verwendeten Ausgangsstoffen um einfach herstellbare Grundchemikalien handelt, die von mehreren unabhängigen Grundstofflieferanten auch in größeren Mengen bezogen werden können. Insgesamt wird durch das erfindungsgemäße Verfahren ein kostengünstigeres, wirtschaftliches und sicheres großtechnisches Verfahren zur Herstellung von herbiziden Wirkstoffen der Formel I zur Verfügung gestellt.

Gegenstand der vorliegenden Erfindung ist
ein Verfahren zur Herstellung von Verbindungen der Formel XV 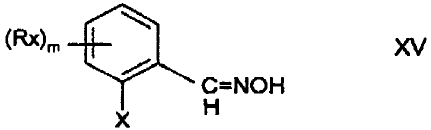 in der die Reste folgende Bedeutung haben:
- X: NO₂, S(O)ₙR_{y};
- Rx, Ry: jeweils ein beliebiger inerter Rest;
- m: 0, 1, 2, 3 oder 4;
- n: 0, 1 oder 2;
umfassend die Umsetzung von Verbindungen der Formel XVI 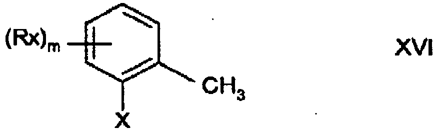 in der die Substituenten die oben angegebene Bedeutung haben, mit einem organischen Nitrit der allgemeinen Formel R-O-NO, wobei R einen aliphatischen oder aromatischen Rest darstellt, unter Einwirkung einer Base, wobei die Umsetzung bei einer Temperatur unterhalb von -20°C in Gegenwart eines dipolar aprotischen Lösungsmittels erfolgt und gegebenenfalls anschließende Umwandlung der Oximgruppe -CH=NOH in Formel XV in die entsprechenden Aldehyde -CHO, Nitrile (-CN) oder Nitriloxide (-CNO), sowie besagtes Verfahren wobei als Lösungsmittel DMF verwendet wird. Gegenstand der vorliegenden Erfindung sind Verbindungen der Formel XV 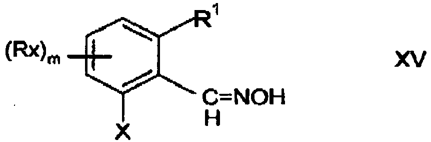 in der die Reste folgende Bedeutung haben:
- x: SO₂R^{y},
- R¹: C₁-C₆-Alkyl
- Rx: Halogen
- Ry: C₁-C₆-Alkyl,
- m: 1, sowie
besagtes Verfahren zur Herstellung von Verbindungen IX 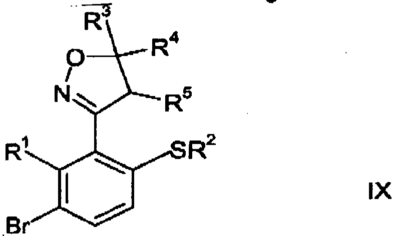 oder X 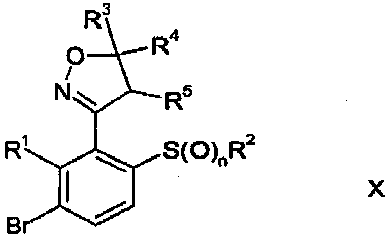 wobei die Substituenten jeweils folgende Bedeutung haben:
- R¹: Wasserstoff, C₁-C₆-Alkyl,
- R²: C₁-C₆-Alkyl,
- R³, R⁴, R⁵: Wasserstoff, C₁-C₆-Alkyl oder R⁴ und R⁵ bilden zusammen eine Bindung,
- n: 1 oder 2.

Beschrieben wird ferner ein Verfahren zur Herstellung der Verbindungen der Formel I 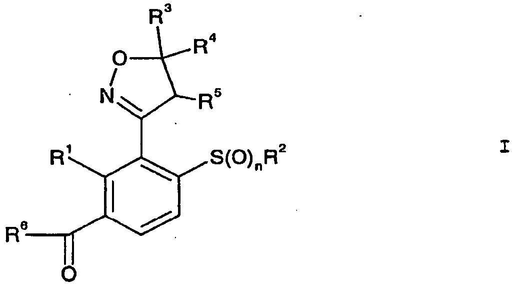 wobei die Substituenten folgende Bedeutung haben:
- R¹: Wasserstoff, C₁-C₆-Alkyl,
- R²: C₁-C₆-Alkyl,
- R³, R⁴, R⁵: Wasserstoff, C₁-C₆-Alkyl oder R⁴ und R⁵ bilden zusammen eine Bindung,
- R⁶: heterocyclischer Ring,
- n: 0, 1 oder 2;
umfassend die Herstellung einer Zwischenverbindung der Formel VI, 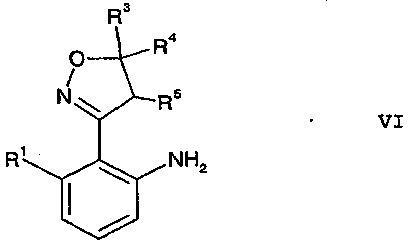 in der R¹, R³- R⁵ die oben angegebene Bedeutungen haben.

Verbindungen der Formel VI werden in anschließenden Reaktionsschritten in die entsprechenden 3-Brom substituierten Verbindungen (Brombenzol-Derivate) überführt und die Aminogruppe am Phenylring in eine Sulfonylgruppe umgewandelt, wobei Verbindungen der Formel X resultieren:

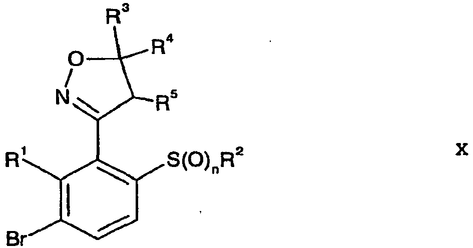

Die Verbindungen der Formel X (3-(4,5-dihydroisoxazol-3-yl)-brom-benzole) stellen wertvolle Zwischenverbindungen zur Herstellung von Wirkstoffen der Formel I dar. Insbesondere liefert das Verfahren die Verbindungen I im letzten Reaktionsschritt in guter Ausbeute. Die Verbindungen I eignen sich beispielsweise, wie in der WO 96/26206 und WO 97/35850 beschrieben, als Pflanzenschutzmittel, insbesondere als Herbizide.

Verbindungen der Formel I bzw. die hierzu erforderlichen Zwischenverbindungen, insbesondere Verbindungen der Formel VI oder X, können durch die Kombination einer oder mehrerer der folgenden Verfahrensstufen a) - g) vorteilhaft hergestellt werden:
a) Umsetzung einer Nitro-o-methyl-phenylverbindung der Formel II 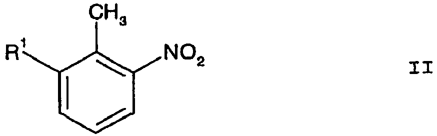 in der der Rest R¹ die vorgenannte Bedeutung hat, mit einem organischen Nitrit R-ONO unter Einwirkung einer Base zum Oxim der Formel III 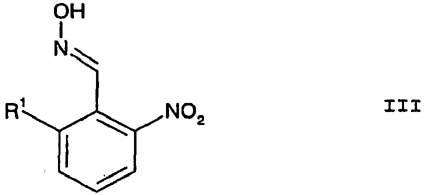 in der der Rest R¹ die vorgenannte Bedeutung hat;
b) Cyclisierung des Oxims der Formel III mit einem Alken der Formel IV 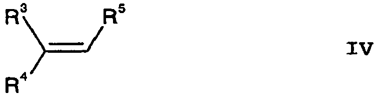 in der R³ bis R⁵ die in Anspruch 1 genannten Bedeutungen haben, in Gegenwart einer Base zum Isoxazol der Formel V 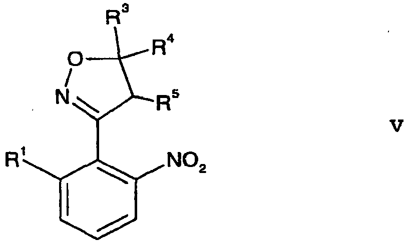 in der R¹, R³ bis R⁵ die in Anspruch 1 genannten Bedeutungen haben;
c) Reduktion der Nitrogruppe in Gegenwart eines Katalysators zum Anilin der Formel VI 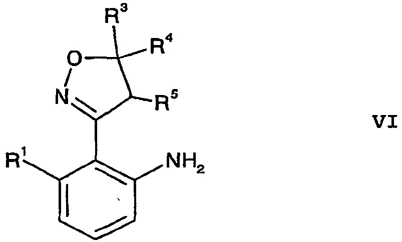 in der R¹, R³ bis R⁵ die in Anspruch 1 genannten Bedeutungen haben;
d) Umsetzung des Anilins der Formel VI mit einem Dialkyldisulfid der Formel VII

   R²-S-S-R² VII

   in Gegenwart eines organischen Nitrits R-ONO und gegebenenfalls eines Katalysators zum Thioether der Formel VIII 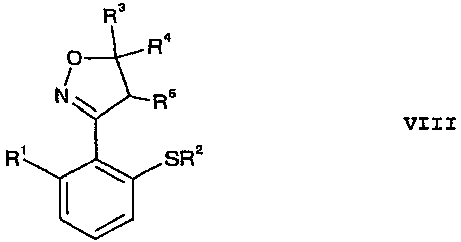 in der R¹ bis R⁵ die in Anspruch 1 genannten Bedeutungen haben;
e) Bromierung des Thioethers der Formel VIII mit einem Bromierungsmittel zum Bromthioether der Formel IX 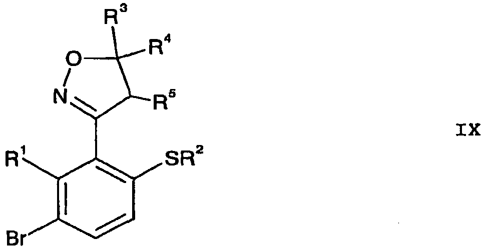 in der R¹ bis _{R}⁵ die in Anspruch 1 genannten Bedeutungen haben;
f) Oxidation des Bromthioethers der Formel IX mit einem Oxidationsmittel zu den Isoxazolen der Formel X 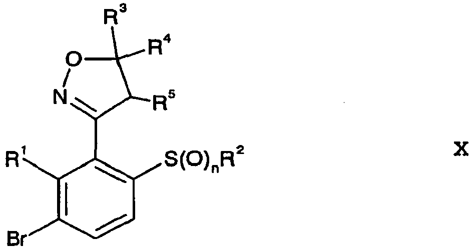 wobei n die Zahlen 1 oder 2 bedeutet,
g) gegebenenfalls Umsetzung des Isoxazolins der Formel X mit einer Verbindung der Formel R⁶-OH (XI) in Gegenwart von Kohlenmonoxid, einem Katalysator und einer Base zu den Verbindungen der Formel I.

Das Verfahren zur Herstellung von Verbindungen X umfaßt im wesentlichen eine oder mehrere der Verfahrensstufen a)-f) bzw. im Falle der Verbindungen I eine oder mehrere der Verfahrensstufen a)-g). Bevorzugt kommen solche Reaktionsabläufe in Frage, die entweder eine der Verfahrensstufen a) oder d) bzw. auch beide Stufen a) und d) umfassen.

C₁-C₆-Alkyl bzw. C₁-C₄-Alkyl bedeutet in allen Fällen eine geradkettige oder verzweigte Alkylgruppen mit 1 - 6 bzw. 1 - 4 C-Atomen, wie z.B. Methyl, Ethyl, n-Propyl, iso-Propyl, n-Butyl, isoButyl, n-Pentyl oder n-Hexyl. Analoges gilt für die C₁-C₆-Alkoxygruppe.

R¹ bedeutet bevorzugt eine Alkylgruppe, insbesondere Methyl, Ethyl, i-Propyl, n-Propyl oder n-Butylgruppe.

R³, R⁴ oder R⁵ bedeuten bevorzugt Wasserstoff. R⁴ und R⁵ können gemeinsam auch eine Bindung darstellen, so daß die entsprechenden Isoxazol-Derivate resultieren. In diesem Fall ist R³ bevorzugt Wasserstoff.

In der Definition von R⁶ bedeutet "heterocyclischer Ring" einen gesättigten, ungesättigten oder teilweise ungesättigten Heterocyclus mit einem, zwei oder drei Sauerstoff-, Schwefel- oder Stickstoffatomen. Bevorzugt sind Heterocyclen mit zwei Stickstoffatomen. Inbesondere bedeutet R⁶ einen Pyrazolrest, wie in WO 98/31681 näher beschrieben. Bevorzugt handelt es sich hierbei um ein in 4-Stellung verknüpftes Pyrazol, das gegebenenfalls durch weitere Reste substituiert sein kann, die unter den gewählten Reaktionsbedingungen chemisch inert sind. Als derartige Substituenten des Pyrazols kommen beispielsweise die folgenden Gruppen in Frage: Hydroxy, Oxo, Sulfonyloxy, C₁-C₆-Alkyl oder C₁-C₆-Alkoxy, insbesondere in 1-Position C₁-C₄-Alky1. Besonders bevorzugt bedeutet R⁶ die Gruppe 1-Alkyl-5-hydroxy-pyrazol-4-yl, insbesondere 1-Methyl-5-hydroxy-pyrazol-4-yl; 1-Ethyl-5-hydroxypyrazol-4-yl.

Das Verfahren eignet sich insbesondere zur Herstellung der folgenden Verbindungen der Formel I:
1-Methyl-4-(3-(4,5-dihydroisoxazol-3-yl)-2-methyl-4-methylsulfonyl-benzoyl)-5-hydroxypyrazol,
1-Ethyl-4-(3-(4,5-dihydroisoxazol-3-yl)-2-methyl-4-methylsulfonyl-benzoyl)-5-hydroxypyrazol,
1-Methyl-4-(3-(4,5-dihydroisoxazol-3-yl)-2-ethyl-4-methylsulfonyl-benzoyl)-5-hydroxypyrazol,
1-Methyl-4-(3-(4,5-dihydroisoxazol-3-yl)-2-propyl-4-methylsulfonyl-benzoyl)-5-hydroxypyrazol,
1-Methyl-4-(3-(4,5-dihydroisoxazol-3-yl)-2-butyl-4-methylsulfonyl-benzoyl)-5-hydroxypyrazol.

Bevorzugt Zwischenverbindungen der Formel VI sind folgende Verbindungen:
2-(4,5-Dihydroisoxazol-3-yl)-anilin,
2-(4,5-Dihydroisoxazol-3-yl)-3-methylanilin,
2-(4,5-Dihydroisoxazol-3-yl)-3-ethylanilin,
2-(Isoxazol-3-yl)-anilin,
2-(Isoxazol-3-yl)-3-methylanilin,
2-(Isoxazol-3-yl)-3-ethylanilin.

Bevorzugt Zwischenverbindungen der Formel X sind folgende Verbindungen:
3-(3-Brom-2-methyl-6-methylsulfonylphenyl)-4,5-dihydroisoxazol,
3-(3-Chlor-2-methyl-6-methylsulfonylphenyl)-4,5-dihydroisoxazol,
3-(3-Brom-6-methylsulfonylphenyl)-4,5-dihydroisoxazol,
3-(3-Brom-2-ethyl-6-methylsulfonylphenyl)-4,5-dihydroisoxazol,
3-(3-Brom-2-isopropyl-6-methylsulfonylphenyl)-4,5-dihydroisoxazol,
3-(3-Brom-2-methyl-6-ethylsulfonylphenyl)-4,5-dihydroisoxazol,
3-(3-Brom-2-methyl-6-propylsulfonylphenyl)-4,5-dihydroisoxazol,
3-(3-Brom-2-methyl-6-butylsulfonylphenyl)-4,5-dihydroisoxazol,
3-(3-Brom-2-methyl-6-pentylsulfonylphenyl)-4,5-dihydroisoxazol,
3-(3-Brom-2-methyl-6-hexylsulfonylphenyl)-4,5-dihydroisoxazol.

Eine mögliche Reaktionsabfolge bis zur Herstellung der Verbindungen X ist anhand des folgenden Übersichtsschemas zusammengestellt: 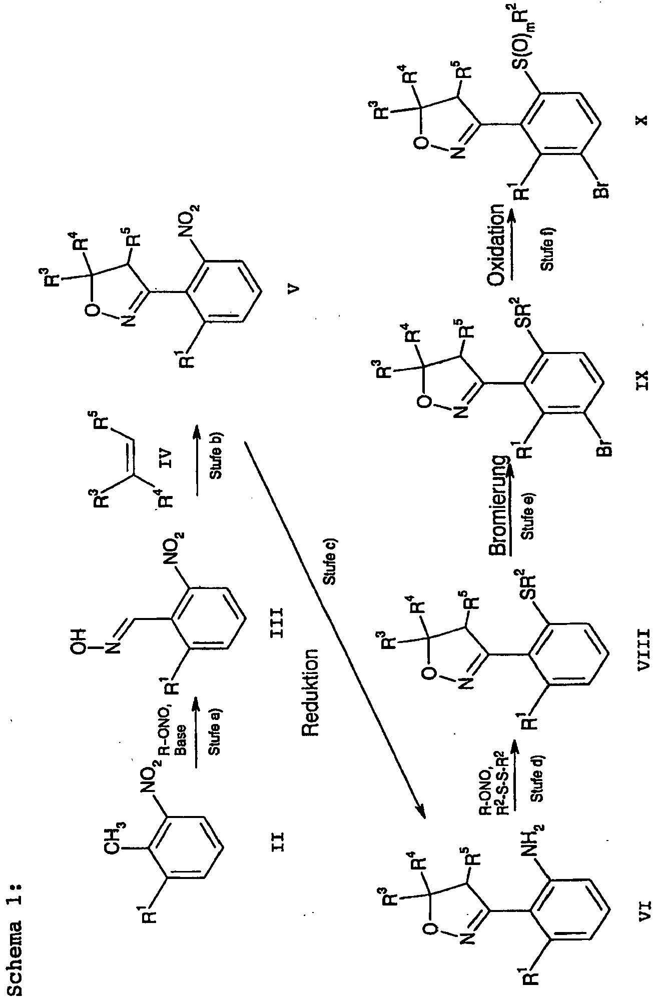 Im folgenden werden die einzelnen Reaktionsstufen näher erläutert.

### 1. Stufe a)

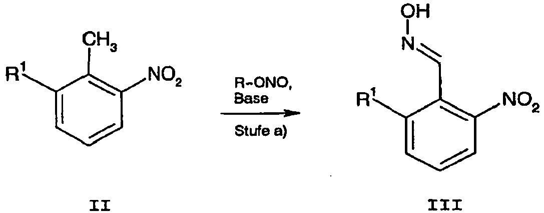

Die Umsetzung erfolgt z.B. unter den folgenden Bedingungen: Als Lösungsmittel werden dipolare aprotische Lösungsmittel verwendet, beispielsweise N,N-Dialkylformamid, N,N-Dialkyl-acetamid, N-Methylpyrrolidon (NMP), bevorzugt: Dimethylformamid (DMF) oder NMP. Die Temperatur beträgt -60°C bis Raumtemperatur; bevorzugt -50 bis -20°C. Um einen ausreichenden niedrigen Schmelzpunkt des Lösungsmittelsystems zu erreichen, können auch Mischungen von Lösungsmitteln, wie z.B. mit THF, eingesetzt werden. Als organische Nitrite R-ONO werden Alkylnitrite (R = Alkyl), bevorzugt n-Butylnitrit oder (iso)Amylnitrit eingesetzt. Als Basen können verwendet werden: MOAlkyl, MOH, RMgX (M = Alkalimetall); bevorzugt Kaliummethylat (KOMe), Natriummethylat (NaOMe) oder Kalium-tert.-Butylat (KOtbutylat). Bei Einsatz von Natriumbasen kann gegebenenfalls 1 - 10 mol-% Amylalkohol zugesetzt werden. Die stöchiometrischen Verhältnisse sind beispielsweise wie folgt: 1 - 4 Equivalente Base, 1 - 2 Equivalente R-ONO; bevorzugt: 1,5 - 2,5 Equivalente Base und 1 - 1,3 Equivalente R-ONO.

Die Zugabe erfolgt beispielsweise nach der folgenden Dosierreihenfolge: a) Nitro-o-xylol und Nitrit vorlegen und Base zudosieren. b) Um Feststoffdosierung der Base zu vermeiden, kann Base in DMF vorgelegt und Nitro-o-xylol/Butylnitrit gleichzeitig zugegeben werden. Die Dosiergeschwindigkeit für die Zugabe der Base erfolgt relativ langsam, so daß die erforderliche Kühlleistung auf ein Minimum reduziert wird. Die Aufarbeitung erfolgt nach einer der folgenden Methoden: a) Ausfällung des Produktes durch Einrühren in Wasser. b) Ausfällen des Produktes durch Zugabe einer ausreichenden Menge an Wasser zum Reaktionsgemisch. Die Produktreinigung erfolgt durch Ausrühren mit Toluol bei Temperaturen von 0 bis 110 °C, bevorzugt bei Raumtemperatur.

### 2. Stufe b)

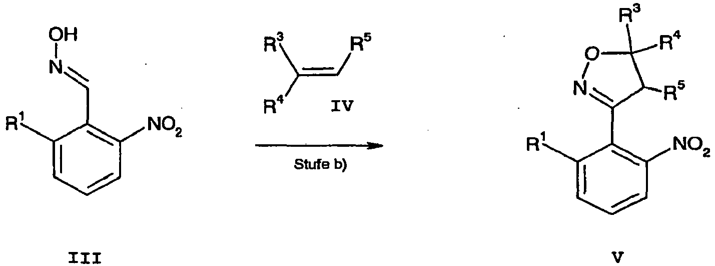

Die Umsetzung erfolgt beispielsweise über die folgenden mechanistischen Zwischenstufen: Überführung des Oxims III in ein aktiviertes Hydroxamsäurederivat, wie z.B. Hydroxamsäurechlorid durch Chlorierung mit einem Chlorierungsmittel, Umwandlung des aktivierten Hydroxamsäurederivates zum Nitriloxid, wie z.B. Überführung des Hydroxamsäurechlorids in Gegenwart einer Base in das Nitriloxid, und anschließende Cycloaddition des Alkens IV an das Nitriloxid.

Bei dieser Umsetzung handelt es sich um ein neues Verfahren zur Herstellung von Isoxazol-Derivaten der Formel V. Dieses Verfahren liefert überraschenderweise die Isoxazoline in sehr guten Ausbeuten. Ferner werden nur wenige Nebenprodukte gebildet, die darüberhinaus auch relativ leicht entfernt werden können. Dadurch ist eine in großtechnischem Umfang einfache Isolierung und Reinigung der Endprodukte möglich, so daß die Isoxazoline in hoher Reinheit und kostenmäßig günstig herzustellen sind. Die Anwendung von bekannten Verfahren zur Herstellung von Isoxazolinen war bisher nachteilig, da die Isoxazoline ausgehend von der Umsetzung der Benzaldoxime nur in unbefriedigenden Ausbeuten erhalten werden konnten. Ferner werden bei den aus dem Stand der Technik bekannten Verfahren oft Alkalihypohalogenid-haltige Lösungen eingesetzt, die zur Bildung von schwerlöslichen und umweltbelastenden Nebenprodukten führen. Das erfindungsgemäße Verfahren ist dadurch gekennzeichnet, daß auf den Einsatz von Alkalihypohalogenidhaltigen Lösungen verzichtet werden kann, und das Verfahren somit im wesentlichen frei von Alkalihypohalogeniden ist.

Die Herstellung der Isoxazoline erfolgt beispielsweise nach folgender Methode: Zunächst wird Hydroxamsäurechlorid gebildet, das in einem zweiten Schritt mit einem Alken unter Zudosieren von Base und ggf. bei Überdruck cyclisiert wird. Diese Einzelschritte können vorteilhaft auch in einer "Eintopfreaktion" zusammengefaßt werden. Dazu wird einem Lösungsmittel gearbeitet, in dem beide Teilschritte ablaufen, z.B. Carbonsäureester wie Essigsäureethylester, Chlorbenzol oder Acetonitril.

Die Herstellung von Hydroxamsäurechloriden mit N-Chlorsuccinimid in DMF ist literaturbekannt (Liu et al., J.Org. Chem. 1980, 45: 3916-3918). Allerdings findet sich auch der Hinweis, daß o-Nitrobenzaldoxime nur mit schlechten Ausbeuten durch Chlorierung in die Hydroxamsäurechloride zu überführen sind (Chiang, J.Org. Chem. 1971, 36: 2146-2155). Als Nebenreaktion ist die Benzalchloridbildung zu erwarten. Nach dem oben beschriebenen Verfahren wurden überraschenderweise Bedingungen gefunden, die es erlauben, die gewünschten Hydroxamsäurechloride in ausgezeichneten Ausbeuten herzustellen. Vorteilhaft ist insbesondere die Verwendung des preisgünstigen Chlors.

Die Umsetzung erfolgt z.B. unter folgenden Bedingungen: Lösungsmittel: Halogenalkane wie 1,2-Dichlorethan oder Methylenchlorid; Aromaten wie Benzol, Toluol, Chorbenzol, Nitrobenzol, oder Xylol; polar aprotische Lösungsmittel, z.B. N,N-Dialkylformamide, - acetamide, N-Methylpyrrolidon, Dimethylpropylenharnstoff; Tetramethylharnstoff, Acetonitril, Propionitril; Alkohole wie Methanol, Ethanol, n-Propanol oder Isopropanol; Carbonsäuren, wie Essigsäure oder Propionsäure; Carbonsäureester, wie Essigsäureethylester. Bevorzugt werden folgende Lösungsmittel verwendet: Essigsäure, Methanol, Ethanol, 1,2-Dichlorethan, Methylenchlorid oder Chlorbenzol oder Essigsäureethylester. Die Umsetzung erfolgt bei Temperaturen von - 40 °C bis 100°C; bevorzugt -10 bis 40°C bzw. 0 bis 30 °C. Als Halogenierungsmittel können verwendet werden: N-Chlorsuccinimid, elementares Chlor, bevorzugt Chlor. Die stöchiometrischen Verhältnisse betragen beispielsweise 1-3 Equivalente Halogenierungsmittel, bevorzugt 1-1,5 Equivalente. Die Dosierung erfolgt im Falle von Chlor durch Einleiten, bei N-Chlorsuccinimid (NCS) als Feststoff oder ggf. in einem geeigneten Lösungsmittel.

Die Aufarbeitung erfolgt beispielsweise nach folgendem Schema: a) Ohne Reinigung. Die Lösung wird weiter eingesetzt; b) Lösungsmitteltausch durch Abdestillieren des Lösungsmittels; c) Zugabe von Wasser und Extraktion des Hydroxamsäurechlorids mit einem geeigneten Lösungsmittel.

Durch Zugabe von Basen werden aus den Hydroxamsäurechloriden die Nitriloxide gebildet. Da diese unbeständig sind, bestand das zu lösende Problem darin, solche Bedingungen zu finden, die es erlauben, die Nitriloxide zu stabilisieren und zu den gewünschten Produkten umzusetzen. Diese Aufgabe wurde überraschenderweise gelöst, wenn folgenden Reaktionsbedingungen gewählt werden: Als Lösungsmittel werden verwendet: Halogenalkane, wie 1,2-Dichlorethan oder Methylenchlorid; Aromaten, wie Benzol, Toluol, Chorbenzol, Nitrobenzol oder Xylol; polar aprotische Lösungsmittel, z.B. N,N-Dialkylformamide, - acetamide, N-Methylpyrrolidon, Dimethylpropylenharnstoff; Tetramethylharnstoff, Acetonitril, Propionitril, Carbonsäureester wie Essigsäureethylester. Bevorzugt werden verwendet: 1,2-Dichlorethan, Methylenchlorid, Toluol, Xylol, Essigsäureethylester oder Chlorbenzol.

Die Temperaturen für die Umsetzung betragen 0 °C bis 100°C, bevorzugt 0 bis 50°C oder 0 bis 30 °C.

Als Basen werden eingesetzt: Tertiäre Amine, z.B. Triethylamin, cylische Amine wie N-Methylpiperidin oder N,N'-Dimethylpiperazin, Pyridin, Alkalicarbonate, z.B. Natriumcarbonat oder Kaliumcarbonat, Alkalihydrogencarbonate, z.B. Natriumhydrogencarbonat oder Kaliumhydrogencarbonat, Erdalkalicarbonate, z.B. Kalziumcarbonat, Alkalihydroxide, z.B. Natriumhydroxid oder Kaliumhydroxid. Bevorzugt werden verwendet: Triethylamin, Natriumcarbonat, Natriumhydrogencarbonat oder Natriumhydroxid.

Die stöchiometrischen Verhältnisse betragen beispielsweise 1-3 Equivalente Base, bevorzugt 1-1,5 Equivalente; 1-5 Equivalente Alken, bevorzugt 1-2 Equivalente. Die Dosierung erfolgt bevorzugt unter Alken-Überdruck durch langsame Zugabe der Base. Die Umsetzung erfolgt bei Normaldruck bis 10 atm, bevorzugt 1 - 6 atm Normaldruck.

### 3. Stufe c)

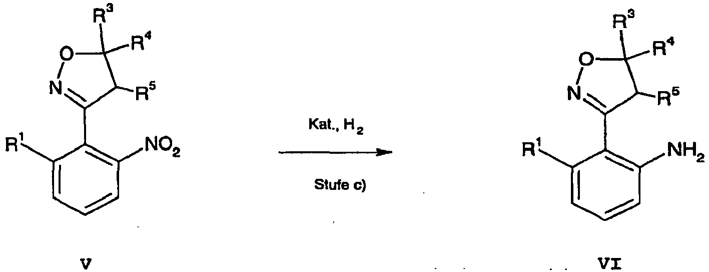

Bei dieser Reaktion handelt es sich um eine neue chemoselektive Hydrierung einer Nitrogruppe neben einem Isoxazolin, die bisher nicht bekannt war. Überraschenderweise wurde gefunden, daß unter den gewählten Reaktionsbedingungen die N-O-Bindung des Isoxazolinringes nicht gespalten wird. Die katalytische Hydrierung von aromatischen Nitroverbindungen zu den Anilinen ist seit langer Zeit bekannt (s. Houben-Weyl, Bd IV/1c, S 506 ff). Auf der anderen Seite ist auch bekannt, daß die N-O-Bindung von Isoxazolinen durch katalytische Hydrierung, z.B. mit Raney-Nickel (Curran et.a1., Synthesis 1986, 312-315) oder Palladium (Auricchio et.al., Tetrahedron, 43, 3983-3986, 1987) als Katalysator gespalten werden kann.

Die Umsetzung erfolgt beispielhaft unter den folgenden Bedingungen: Als Lösungsmittel eignen sich Aromaten wie Benzol, Toluol, Xylol; polar aprotische Lösungsmittel, z.B. N,N-Dialkylformamide, - acetamide, N-Methylpyrrolidon, Dimethylpropylenharnstoff; Tetramethylharnstoff, Carbonsäureester wie Essigsäureethylester, Ether wie Diethylether oder Methyltert.Butylether, cyclische Ether wie Tetrahydrofuran oder Dioxan; Alkohole wie Methanol, Ethanol, n-Propanol, oder Isopropanol, Carbonsäuren wie Essigsäure oder Propionsäure. Bevorzugt werden folgende Lösungsmittel verwendet: Essigester, Toluol, Xylol, Methanol. Die Umsetzung erfolgt bei Temperaturen von -20 °C bis 100°C; bevorzugt 0 bis 50°C, bes. bevorzugt 0 bis 30 °C. Als Katalysator verwendet man einen Platin- oder Palladiumkatalysator auf einem Aktivkohleträger bei einem Gehalt 0,1 bis 15 Gew%, bezogen auf den Aktivkohleträger. Bei Verwendung von Palladiumkatalysator kann dieser mit Schwefel oder Selen dotiert sei, um eine bessere Selektivität zu erhalten. Bevorzugt wird Platin/Aktivkohle oder Palladium/Aktivkohle mit einem Gehalt von Pt oder Pd von 0,5 - 10 Gew.% eingesetzt.

Für die Umsetzung sind die stöchiometrischen Verhältnisse beispielsweise wie folgt: 0,001 bis 1 Gew% Platin oder Palladium bezogen auf die Nitroverbindung; bevorzugt 0,01 bis 1 Gew% Platin. Die Dosierung der Zugabe von Wasserstoff erfolgt kontinuierlich oder diskontinuierlich, bevorzugt diskontinuierlich bei Normaldruck bis 50 atm, bevorzugt Normaldruck bis 10 atm.

Die Aufarbeitung der Reaktionsmischung erfolgt durch Abtrennen des Katalysators durch Filtration. Der Katalysator kann ggf. auch wieder eingesetzt werden. Das Lösungsmittel wird abdestilliert. Für die folgende Umsetzung in der nächsten Verfahrensstufe kann das Produkt direkt ohne weitere Aufreinigung verwendet werden. Bei Bedarf kann das Produkt gegebenenfalls auch weiter aufgereinigt werden. Die Produktreinigung erfolgt beispielsweise nach dem folgenden Schema: Wenn erforderlich kann das Anilin durch Aufnehmen in verdünnter Mineralsäure, z.B. wäßriger Salzsäure oder verdünnter Schwefelsäure, Extraktion mit einem geeigneten organischen Extraktionsmittel, z. B. Halogenalkane wie 1,2-Dichlorethan oder Methylenchlorid, Aromaten wie Benzol, Toluol, Chorbenzol oder Xylol, Ether wie Diethylether oder Methyl-tert.Butylether, Carbonsäureester wie Essigsäureethylester gereinigt werden und durch eine Base wieder freigesetzt werden.

### 4. Stufe d)

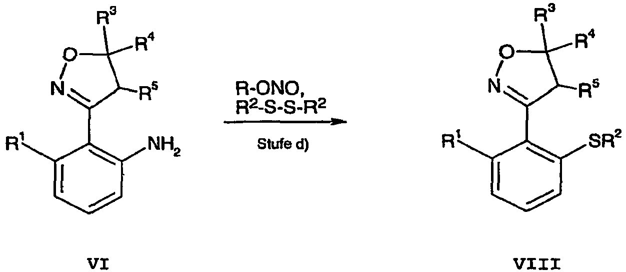

Die Umsetzung erfolgt unter folgenden Bedingungen: Als Lösungsmittel werden beispielsweise verwendet: Halogenalkane wie 1,2-Dichlorethan oder Methylenchlorid, Aromaten wie Benzol, Toluol, Chorbenzol, Nitrobenzol, oder im Überschuß des Dialkyldisulfids als Lösungsmittel. Bevorzugt wird ein Überschuß des Dialkyldisulfids als Lösungsmittels eingesetzt. Die Temperatur für die Umsetzung beträgt 40 °C bis 150°C; bevorzugt 50 bis 100°C, bes. bevorzugt 60 bis 90 °C. Als Reagenz werden organische Nitrite (R-ONO), wie z.B. Alkylnitrite, eingesetzt, bevorzugt n-Butylnitrit, (iso)Amylnitrit oder tert-Butylnitrit. R bedeutet hierbei einen beliebigen, organischen und chemisch inerten Rest, der die eigentliche Reaktion nicht beeinflußt. R ist beispielsweise eine C₁-C₆-Alkyl oder C₂-C₆-Alkenylgruppe.

Bei der Umsetzung der Verbindungen sind die stöchiometrischen Verhältnisse beispielsweise wie folgt: 1 - 3 Equivalente Alkylnitrit, bevorzugt 1 - 1,5 Equ. Alkylnitrit. Als Katalysatoren können verwendet werden: Kupferpulver, elementares Kupfer in anderer Form, wie z.B. Späne, Draht, Granulat, Schrot, Stäbe; Kupfer-I-Salze, z.B. Kupfer-I-Chlorid, Kupfer-I-Bromid oder Kupfer-I-Jodid, Kupfer-II-Salze, oder elementares Jod, besonders bevorzugt Kupferpulver. Bei der Durchführung der Reaktion im Lösungsmittel werden 1-3 Equivalente Dialkyldisulfid, bevorzugt 1-2 Equivalente, eingesetzt. In einer bevorzugten Ausführungsform wird das Dialkyldisulfid als Lösungsmittel im Überschuß eingesetzt und anschließend destillativ zurückgewonnen. Für die weitere Umsetzung kann das Produkt ohne weitere Reinigung eingesetzt werden. Gegebenenfalls kann zuvor auch eine Produktreinigung erfolgen durch Destillation oder Kristallisation mit Hilfe von geeigneten Lösungsmitteln, wie z.B. aus Diisopropylether.

### 5. Stufe e)

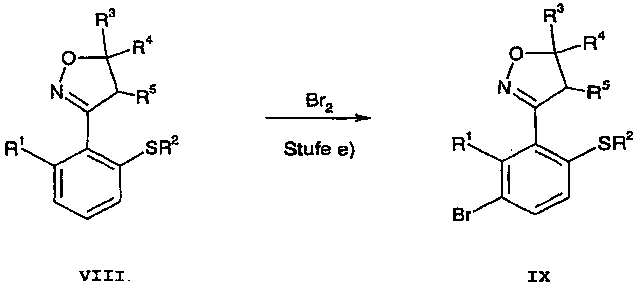

Die Bromierung erfolgt analog zu der in WO 98/31676 beschriebenen Methode. Als Lösungsmittel ist Essigsäure vorteilhaft.

### 6. Stufe f)

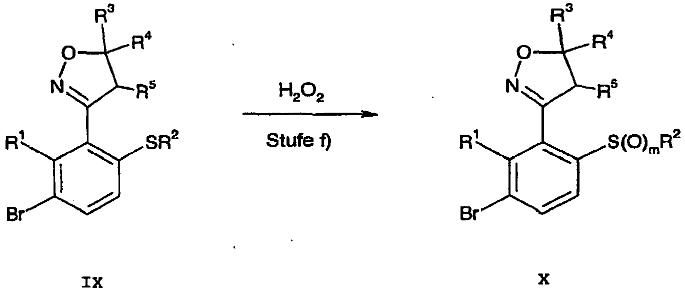

Die Oxidation erfolgt analog zu der in WO 98/31676 beschriebenen Methode (vgl. S. 8 Zeile 32 bis S. 11, Zeile 25).

### 7. Stufe g)

Die gegebenenfalls sich anschließende Umsetzung der Verbindung der Formel X zu Verbindungen der Formel I erfolgt durch Zugabe von R⁶-OH (XI) in Gegenwart von Kohlenmonoxid und einem geeigneten Katalysator und einer Base. Für den Fall, daß R⁶ einen ggf. substituierten Pyrazol- bzw. Pyrazolonring darstellt, erfolgt die Umsetzung vorzugsweise mit palladiumhaltigen Katalysatoren, wie z.B. Pd(0)-Katalysator oder Bis-triphenylphosphin-Palladium(II)-chlorid.

Bei dem in Stufe g) genannten Verfahren handelt es sich um ein neues und vorteilhaftes Verfahren zur Herstellung von Verbindungen der Formel I, die ausgehend von Halogenphenylderivaten X durch Acylierung bzw. Carboxylierung mit Hydroxy-substituierten Heterocyclen der Formel R⁶-OH (XI) erhalten werden.

Aus EP-A 344 775 ist ein Verfahren zur Herstellung von 4-Benzoyl-5-hydroxypyrazolen in einer Stufe bekannt, wobei die Synthese ausgehend von Brombenzolen und 5-Hydroxypyrazolen in Gegenwart von Kohlenmonoxid, Base und Katalysator erfolgt. Der Benzoylrest der Zielmoleküle kann in 3-Stellung die folgenden Substituenten tragen: Alkoxycarbonyl, Alkoxy, Alkoxymethyl. Diese Substituenten gelten chemisch als relativ stabil bzw. inert und erlauben den Ausführungsbeispielen zufolge drastische Reaktionsbedingungen. Hingegen ist die Herstellung von Benzoyl-5-hydroxypyrazolen, die in 3-Stellung weniger stabile Substituenten tragen, wie dies z.B. für den Isoxazol- bzw. Isoxazolinrest der Fall ist, in der EP 0 344 775 im Hinblick auf die drastischen Reaktionsbedingungen nicht beschrieben. Der Isoxazol- bzw. Isoxazolinrest gilt insbesondere hinsichtlich seiner Redoxeigenschaften als ein sehr empfindlicher Rest. Ein Nachteil des aus EP-A 344 775 bekannten Verfahrens liegt weiterhin darin begründet, daß das 5-Hydroxypyrazol stets in großem Überschuß eingesetzt wird.

Das Verfahren wird im folgenden anhand des Bespiels mit R⁶ = Pyrazol (XI.a) als Heterocyclus näher erläutert. Grundsätzlich können jedoch auch andere heterocyclische Verbindungen, wie eingangs definiert, verwendet werden.

Bevorzugt wird das Verfahren so durchgeführt, daß ein Hydroxypyrazol der Formel XI.a 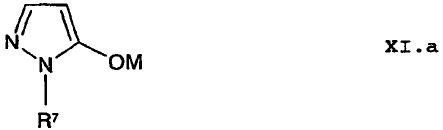 in der R⁷ C₁-C₆-Alkyl darstellt und M für Wasserstoff oder ein Alkalimetallatom, vorzugsweise Natrium oder Kalim, steht, und ein Brombenzol der Formel X, 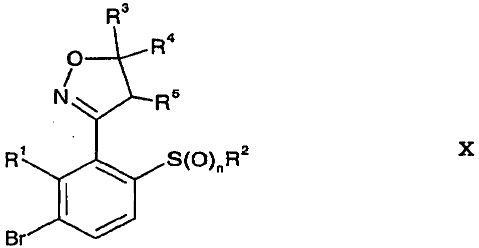 in der R¹ bis R⁵ die vorgenannte Bedeutung besitzen, in Gegenwart von Kohlenmonoxid, einem Palladiumkatalysator, gegebenenfalls mindestens einem Moläquivalent eines Kaliumsalzes und gegebenenfalls mindestens einem Moläquivalent eines tertiären Amins der Formel XIII,

N(Rₐ)₃ XIII

in der einer der Reste Rₐ für Phenyl oder Naphthyl stehen kann und die übrigen Reste Rₐ C₁-C₆-Alkyl bedeuten, bei Temperaturen von 100 bis 140°C und einem Druck von 1 bis 40 kg/cm² miteinander zur Reaktion gebracht werden.

In einer bevorzugten Ausführungsform des Verfahrens werden das 5-Hydroxypyrazol XI.a und das Brombenzolderivat X in einem Molverhältnis von 1 bis 2 eingesetzt.

Bevorzugt werden als 5-Hydroxyprazole XI.a solche Verbindungen eingesetzt, in denen R⁷ C₁-C₆-Alkyl, insbesondere Methyl oder Ethyl, bedeutet.

Die als Ausgangsmaterialien verwendeten 5-Hydroxypyrazole (bzw. Pyrazolinone) der Formel XI.a sind bekannt und können nach an sich bekannten Verfahren hergestellt werden (vgl. EP-A 240 001, WO 96/26206 und J. Prakt. Chem. 315 (1973) S. 382).

Das 5-Hydroxypyrazol XI.a wird in der Regel in Bezug auf das Brombenzolderivat X äquimolar oder im Überschuß eingesetzt. Aus Gründen der Wirtschaftlichkeit ist es sinnvoll, einen größeren Überschuß an 5-Hydroxypyrazol zu vermeiden. Unter den erfindungsgemäßen Reaktionsbedingungen wird beim stöchiometrischen Ansatz die gleiche Ausbeute erhalten wie bei Verwendung eines Überschusses an 5-Hydroxypyrazol. Dies war überraschend, wird doch in sämtlichen Beispielen des in EP-A 344 775 beschriebenen Verfahrens mit einem großen Überschuß an 5-Hydroxypyrazol gearbeitet. Im erfindungsgemäßen Verfahren wird vorzugsweise ein Molverhältnis von 5-Hydroxypyrazol zu Brombenzol von 1 bis 2 und insbesondere bevorzugt von 1,0 bis 1,2 eingestellt.

Oberhalb von 140°C tritt Zersetzung ein, unterhalb von 100°C kommt die Reaktion zum erliegen. Es wird daher im allgemeinen in einem Temperaturbereich von 100 bis 140°C und vorzugsweise von 110 bis 130°C gearbeitet.

Überraschenderweise wurde gefunden, daß der für die Reaktion üblicherweise erforderliche hohe Druck im Bereich von bis zu 150 kg/cm² (vgl. hierzu Angaben in EP 0 344 775) auf einen Wert von maximal bis zu 40 kg/cm², bevorzugt bis zu 20 kg/cm² oder auch bis zu 10 kg/cm² erniedrigt werden kann, ohne daß dies zu einer negativen Beeinträchtigung der Reaktionsbedingungen, wie Reaktionstemperatur oder Reaktionszeit oder zu einer Ausbeuteverringerung führt. Bevorzugt beträgt der Reaktionsdruck mindestens 3 kg/cm², insbesondere mindestens 5 kg/cm². Beispielhaft kommen folgende Druckbereiche in Frage: 1 - 40 kg/cm², 5 - 20 kg/cm² oder 10 - 20 kg/cm², insbesondere 3 - 10 und besonders bevorzugt 5 - 8 kg/cm².

Diese Erniedrigung des Druckes ist für das großtechnische Herstellungsverfahren ein besonders vorteilhaft, da hierdurch hinsichtlich der verwendeten Druckbehälter geringere Sicherheitsanforderungen zu erfüllen sind. Auf die kostenaufwendige Verwendung von Hochdruckbehältern kann dadurch verzichtet werden. Das in g) beschriebene Herstellungsverfahren zeichnet sich demzufolge an einem höheren Maß an Sicherheit und Wirtschaftlichkeit aus.

Ferner wurde überraschenderweise gefunden, daß die als Katalysator eingesetzten Palladiumverbindungen unter den gewählten Verfahrensbedingungen größtenteils als elementares Palladium anfallen und auf einfache Weise durch Filtration aus der Reaktionsmischung abgetrennt werden können. Ein verfahrens- und kostenmä-βig aufwendiges Konzentrieren der palladium-haltigen Reaktionslösung für die nachträgliche Entsorgung und eventuelle Verbrennung der Rückstände kann somit weitgehend entfallen. Hierdurch sinken die Kosten für das Recycling. Die Porengröße des ausgefallenen Palladiums beträgt 1 - 10 µm, insbesondere 1 - 4 µm. Das so abfiltrierte Palladium kann kostengünstig wieder zu den entsprechenden Palladiumverbindungen, wie z.B. Palladiumchlorid, aufgearbeitet werden, da die Recyclingkosten von der Konzentration des Palladium abhängen.

Als Lösungsmittel für die Umsetzung in Verfahrensstufe g) können Nitrile wie Benzonitril und Acetonitril, Amide wie Dimethylformamid, Dimethylacetamid, Tetra-C₁-C₄-alkylharnstoffe oder N-Methylpyrrolidon und vorzugsweise Ether wie Tetrahydrofuran, Methyl-tert.-butylether dienen. Insbesondere werden Ether wie 1,4-Dioxan und Dimethoxyethan als Lösungsmittel bevorzugt.

Als Katalysatoren eignen sich Palladiumligandkomplexe, in denen das Palladium in der Oxidationsstufe 0 vorliegt, metallisches Palladium, das gegebenenfalls auf einen Träger aufgezogen wurde, und vorzugsweise Palladium(II)salze. Die Umsetzung mit Palladium(II)salzen und metallischem Palladium wird vorzugsweise in Gegenwart von Kompexliganden durchgeführt.

Als Palladium(0)ligandkomplex kommen beispielsweise Tetrakis(triphenylphosphan)palladium in Frage.

Metallisches Palladium ist vorzugsweise auf einen inerten Träger wie beispielsweise Aktivkohle, Siliciumdioxid, Aluminiumoxid, Bariumsulfat oder Calciumcarbonat aufgezogen. Die Reaktion wird vorzugsweise in Gegenwart von Komplexliganden wie beispielsweise Triphenylphosphan durchgeführt.

Als Palladium(II)salze eigenen sich beispielsweise Palladiumacetat und Palladiumchlorid. Bevorzugt wird in Gegenwart von Komplexliganden wie beispielsweise Triphenylphosphan gearbeitet.

Geeignete Komplexliganden für die Palladiumligandkomplexe, bzw. in deren Gegenwart die Umsetzung mit metallischem Palladium oder Palladium(II)salzen vorzugsweise ausgeführt wird, sind tertiäre Phosphane, deren Struktur durch folgende Formeln wiedergegeben wird: 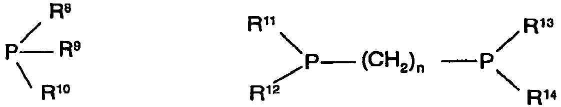 wobei n die Zahlen 1 bis 4 bedeutet und die Reste _{R}⁸ bis _{R}¹⁴ für C₁-C₆-Alkyl, Aryl-C₁-C₂-alkyl oder vorzugsweise Aryl stehen. Aryl steht beispielsweise für Naphthyl und gegebenenfalls substituiertes Phenyl wie beispielsweise 2-Tolyl und insbesondere für unsubstituiertes Phenyl.

Die Herstellung der komplexen Palladiumsalze kann in an sich bekannter Weise ausgehend von kommerziell erhältlichen Palladiumsalzen wie Palladiumchlorid oder Palladiumacetat und dem entsprechenden Phosphanen wie z.B. Triphenylphosphan oder 1,2-Bis(diphenylphosphano)ethan erfolgen. Ein Großteil der komplexen Palladiumsalze ist auch kommerziell erhältlich. Bevorzugte Palladiumsalze sind [(R)(+)2,2'-Bis(diphenylphosphano)-1,1'-bi-naphthyl]palladium(II)chlorid, Bis(triphenylphosphan)palladium(II)acetat und insbesondere Bis(triphenylphosphan)palladium(II)chlorid.

Der Palladiumkatalysator wird in der Regel in einer Konzentration von 0,05 bis 5 Mol%, und bevorzugt 1-3 Mol% eingesetzt.

Für das Verfahren geeignete Amine N(Rₐ)₃ der Formel XIII sind tertiäre Amine wie beispielsweise N-Methylpiperidin, Ethyldiisopropylamin, 1,8-Bisdimethylaminonaphthalin oder insbesondere Triethylamin.

Als Kaliumsalze eignen sich beispielsweise Kaliumphosphat, Kaliumcyanid und insbesondere Kaliumcarbonat. Der Wassergehalt des Kaliumsalzes sollte vorteilhafterweise gering sein. Deshalb wurde das Kaliumcarbonat vor Einsatz in der Regel bei mindestens 150°C getrocknet.

Die Menge an eingesetztem Kaliumsalz beträgt vorteilhafterweise mindestens 1 Moläquivalent. Anderenfalls verlangsamt sich die Reaktion bzw. die intermediär stattfindende Fries-Umlagerung läuft nicht vollständig ab und es werden 0-acylierte Pyrazolderivate erhalten. Bevorzugt werden jeweils 2 bis 4 Moläquivalente und insbesondere bevorzugt 2 Moläquivalente Kaliumsalz bezogen auf das Brombenzol III eingesetzt.

Bevorzugt wird der Reaktionsmischung neben dem Kaliumsalz noch ein Amin N(Rₐ)₃ der Formel XIII, in der einer der Reste Rₐ für Phenyl oder Naphthyl stehen kann und die übrigen Reste Rₐ C₁-C₆-Alkyl bedeuten, zugesetzt. Vorzugsweise werden 1 bis 4 Moläquivalente und insbesondere bevorzugt 2 Moläquivalente des Amins XIII bezogen auf das Brombenzol X eingesetzt.

Zur Aufarbeitung wird die Reaktionslösung in der Regel in Wasser eingetragen. Sofern die Reaktion in einem mit Wasser mischbaren Lösungsmittel wie 1,4-Dioxan durchgeführt wird, kann es von Vorteil sein, das Lösungsmittel zuvor teilweise oder vollständig aus dem Reaktionsmischung gegebenenfalls unter vermindertem Druck zu entfernen. Die wäßrige, alkalische Reaktionsmischung wird von gegebenenfalls festen Bestandteilen abgetrennt und anschließend wird durch Ansäuern mit einer Mineralsäure wie z.B. Salzsäure ein pH von 2,5 bis 4,5, bevorzugt von 3,5 eingestellt, wobei das Wertprodukt nahezu vollständig ausfällt. Insbesondere der Isoxazolinrest ist hydrolyseempfindlich. PH-Werte kleiner 2 sollten in Verfahren zur Herstellung von Benzoylpyrazolen, die diesen Rest aufweisen, vorzugsweise vermieden werden.

Bevorzugt werden für die Acylierung im Verfahrensschritt g) folgende Verfahrensbedingungen gewählt: Lösungsmittel: Dioxan oder Mischungen von Dioxan und Acetonitril. Temperatur: zwischen 110 - 130 °C. Druck: 5 - 8, vorzugsweise etwa 6 kg/cm². Katalysator: Palladium(II)chlorid. Molverhältnis der heterocyclischen Hydroxyverbindungen (wie z.B. 5-Hydroxypyrazol) zu Brombenzol-Derivaten von 1 bis 2 und insbesondere bevorzugt von 1,0 bis 1,2.

In Schema 2 ist eine weitere mögliche Syntheseroute zu den Brombenzol-Derivaten vom Typ der Formel X aufgeführt.

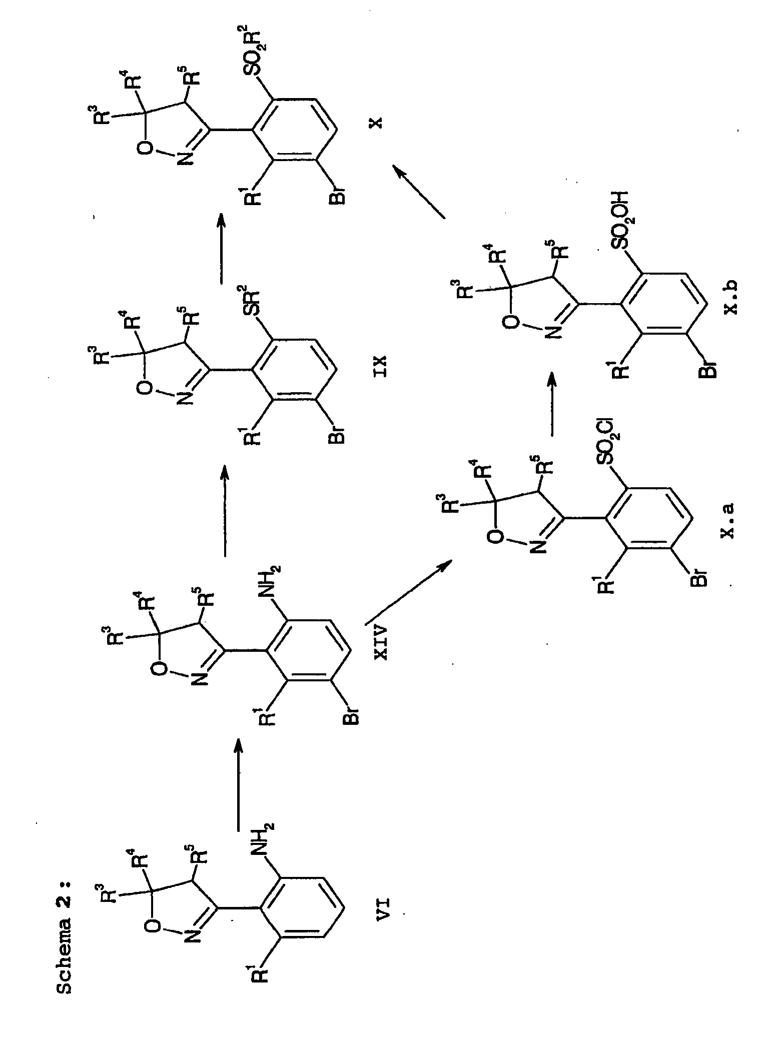

Die Bromierung von Verbindungen der Formel VI erfolgt in ähnlicher Weise wie die direkte Bromierung von Anilinen. Wird Tetrabutylammonium-tribromid als Reagenz eingesetzt, kann in manchen Fällen eine selektive Monobromierung in para-Position zur AminFunktion erreicht werden (Berthelot et al., Synth. Commun. 1986, 16: 1641). Ein generelles Problem bei solchen Bromierungen ist jedoch die Bildung von mehrfach bromierten Produkten (Bull.Chem.Soc.Jpn. 1988, 61: 597-599). Beispielsweise wird bei der Umsetzung von VI mit Tetrabutylammonium-tribromid in einem Methanol/Wasser-Gemisch und Calciumcarbonat als Base eingesetzt, erhält man ein Produktgemisch, das ca. 25% dibromiertes Nebenprodukt enthält. Die Auftrennung des Produktgemisches ist insbesondere auch dann kritisch, wenn Isoxazol- bzw. Isoxazolinreste als Substituenten vorhanden sind, die unter den gewählten Reaktionsbedingungen hinsichtlich ihrer Redoxeigenschaften als labil gelten.

Es wurden nun Bedingungen gefunden, die es erlauben, das gewünschte Produkt XIV mit guten Ausbeuten ohne die Bildung von höher bromierten Nebenprodukten herzustellen. Nach den erfindungsgemäßen Reaktionsbedingungen wird als Reagenz bevorzugt Tetrabutylammonium-tribromid eingesetzt. Als Lösungsmittel werden Halogenalkane, wie 1,2-Dichlorethan oder Methylenchlorid, Alkohole wie Methanol, Ethanol, n-Propanol, i-Propanol, aliphatische Nitrile wie Acetonitril, bevorzugt, Acetonitril verwendet. Als Base Base findet bevorzugt Kaliumcarbonat Verwendung. Die bromierten Zwischenprodukte XIV können dann auf verschiedenen Wegen in die erfindungsgemäßen Isoxazol-3-yl-brombenzole X umgewandelt werden. Die Stufen zur Herstellung von Verbindungen IX aus XIV bzw. Verbindungen X aus IX können nach den oben bereits angegeben Verfahren hergestellt werden.

Alternativ können aber auch die Aniline zunächst in die Sulfochloride X.c überführt werden (s. Houben-Weyl, Bd IX, S 579-580). Diese können durch Reduktion der Sulfochloride z.B. mit Natriumsulfit, über die Stufe der Sulfinsäuren (s. Houben-Weyl, Bd IX, S 306-307) und anschließende Alkylierung (s. Houben-Weyl, Bd IX, S 231-233) in die Alkylsulfone umgewandelt werden. Die beiden Schritte können vorteilhaft in einer "Eintopfreaktion" zusammengefaßt werden. Der Vorteil dieser Synthese liegt in der Verwendung günstiger Einsatzstoffe zur Einführung der Alkylsulfonylgruppen.

Bei dem in Verfahrensschritt a) des erfindungsgemäßen Verfahrens verwendeten Schritt der Oximierung von substituierten Toluolen handelt es sich um ein neues und vorteilhaftes Verfahren zur Überführung von Toluolderivaten in Benzaldoxime. Dieses Verfahren eignet sich grundsätzlich zur Herstellung von Benzaldoximen der Formel XV 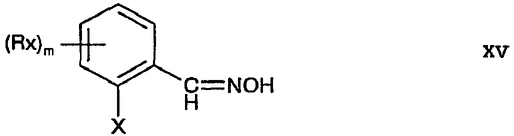 in der die Reste folgende Bedeutungen haben:
- X: N0₂, S(O)ₙRy,
- Rx: ein beiliebiger inerter Rest;
- Ry: ein beiliebiger inerter Rest;
- m: 0, 1, 2, 3 oder 4,
- n: 0, 1 oder 2.

Rx, Ry sind beliebige organische Reste, die gleich oder verschieden sein können und unter den gewählten Reaktionsbedingungen chemisch inert sind. Beispielhaft seien für Rx genannt: Halogen, wie z.B. Chlor, Brom oder Jod; Carboxyl; Carboxamid; N-Alkyl-carboxamide und N,N-Dialkyl-carboxamide; Phenyl; C₁-C₆-Alkyl, wie z.B. Methyl, Ethyl; C₁-C₆-Alkoxy; C₁-C₆-Alkylthio oder andere Reste. Für den Fall, daß m>1 ist, kann Rx jeweils gleich oder verschieden sein. Bevorzugt hat Rx die gleiche Bedeutung wie R¹ und steht ortho-ständig zur Oximgruppe -CH=NOH. Insbesondere bedeutet m die Zahl 2, wobei einer der Substituenten Rx die gleiche Bedeutung wie R¹ hat, und der zweite Substituent Rx ein Halogenatom darstellt, das bevorzugt in meta-Stellung zur Oximgruppe steht. Ry bedeutet bevorzugt C₁-C₆-Alkyl, z.B. Methyl, Ethyl, Propyl.

Bevorzugte Verbindungen XV sind solche, in denen X die Gruppe S0₂-Ry bedeutet, und m die Zahl 2 ist. In diesem Fall ist einer der Reste Rx bevorzugt Halogen (z.B. Brom oder Chlor) und steht in meta-Stellung zur Oximgruppe. Der zweite Rest Rx ist bevorzugt C₁-C₆-Alkyl (z.B. Methyl, Ethyl) und steht in ortho-Stellung zur Oximgruppe.

Erfindungsgemäß werden Verbindungen der Formel XVI (o-Nitrotoluol oder o-Alkylsulfonyltoluol) 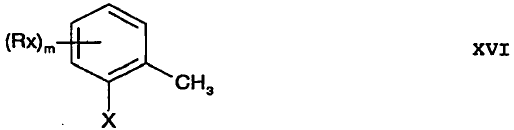 in der die Substituenten die oben angegebene Bedeutung haben, mit einem organischen Nitrit der allgemeinen Formel R-O-NO, wie bereits definiert, unter Einwirkung einer Base umgesetzt.

Die Nitrosierung von o-Nitrotoluol ist in der Literatur beschrieben (Lapworth, J Chem.Soc. 1901, 79: 1265) Allerdings wird auch in dieser frühen Arbeit schon ein dimeres Nebenprodukt erwähnt. Spätere Arbeiten beschreiben dann nur noch die Herstellung von dimeren Produkten unter ähnlichen Reaktionsbedingungen [Das et al., J.Med.Chem. 1970, 13: 979). Die Nachstellung des in der Literatur beschriebenen Versuches mit o-Nitrotoluol zeigt, daß sich in geringer Menge tatsächlich das 2-Nitrobenzaldoxim bildet.

Die Übertragung der beschriebenen Bedingungen auf das 3-Nitro-o-xylol liefert ausschließlich Dimeres XVIII.

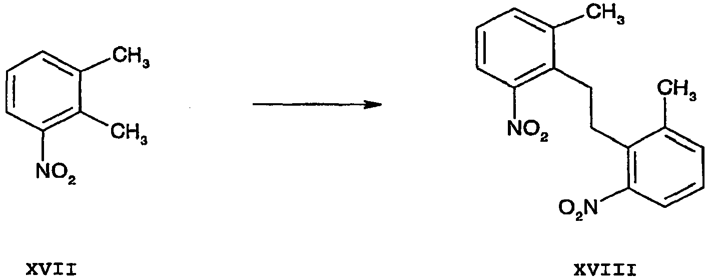

Auch für Michael-Additionen, die unter ähnlichen Bedingungen ablaufen, findet sich in der Literatur der Hinweis, daß sie an 3-Nitro-o-xylol nicht gelingen (Li, Thottathil, Murphy, Tetrahedron Lett. 1994, 36: 6591). Es ist daher nach den Vorbeschreibungen unerwartet, daß aus 6-substituierten 2-Nitrotoluolen Benzaldoxime in ausgezeichneten Ausbeuten herzustellen sind. Außerdem wurde überraschend gefunden, daß Alkylsulfonate (X = SO₂Ry) unter vergleichbaren Bedingungen ebenfalls an der o-ständigen Methylgruppe oximiert werden können. Die nach dem Verfahren der Erfindung hergestellten Verbindungen stellen wichtige Zwischenprodukte für die Herstellung von Wirkstoffen in Pflanzenschutzmittel dar (WO 98/31681).

Die Umsetzung erfolgt unter folgenden Bedingungen: Als Lösungsmittel werden verwendet: Dipolar aprotische Lösungsmittel, z.B. N,N-Dialkylformamid, N,N-Dialkylacetamide, N-Methyl-pyrrolidon, bevorzugt: DMF, NMP. Die Temperatur beträgt - 60 °C; bevorzugt - 50 bis - 20 °C. Als Nitrit oder Alkylnitit wird bevorzugt n-Butylnitrit, (iso)Amylnitrit verwendet. Als Basen eignen sich: (M = Alkalimetall): MOAlkyl, MOH, RMgX; bevorzugt KOMe, NaOMe, KOtbutylat. Beim Einsatz von Natriumbasen werden bevorzugt 1 - 10 mol-% Amylalkohol zugesetzt. Die Stöchiometrie beträgt wie folgt: 1 - 4 Equivalente Base, 1 - 2 Equivalente RONO; bevorzugt: 1,5 - 2,5 Equ. Base, 1 - 1,3 Equ. RONO (i.e. ein organisches Nitrit). Zur Dosierreihenfolge: a) Nitro-o-xylol und Nitrit vorlegen und Base zudosieren. b) Um Feststoffdosierung der Base zu vermeiden, kann Base in DMF vorgelegt und Nitro-o-xylol/Butylnitrit gleichzeitig zugegeben werden. Eine verlängerte Dosierzeit der Base ist vorteilhaft zur Verminderung der erforderlichen Kühlleistung.

Die Aufarbeitung erfolgt beispielsweise wie folgt: a) Ausfällung durch Einrühren in Wasser/Säure. b) Ausfällen durch Zugabe einer ausreichenden Menge an Wasser/Säure. Als Säure können Mineralsäuren wie Schwefelsäure, Salzsäure oder Phosphorsäure oder auch Carbonsäuren wie Essigsäure dienen. Produktreinigung: Durch Ausrühren mit Toluol von 0 bis 110°C, bevorzugt bei Raumtemperatur.

Wird die Reaktion bei höherer Temperatur (-10 bis 0°C) durchgeführt und anschließend bei Raumtemperatur nachgerührt, erhält man nach Aufarbeitung direkt die Benzonitrile. Ferner kann aus den Benzaldoximen der Formel XV in Gegenwart eines sauren Katalysators und eines aliphatischen Aldehyds, z.B. wässrige Formaldehydlösung, die Aldehydfunktion freisetzt werden. Als Lösungsmittel eignen sich Halogenalkane, wie 1,2-Dichlorethan oder Methylenchlorid, Aromaten wie Benzol, Toluol, Chorbenzol, Nitrobenzol, oder Xylol, polar aprotische Lösungsmittel, z.B. N,N-Dialkylformamide, - acetamide, N-Methylpyrrolidon, Dimethylpropylenharnstoff; Tetramethylharnstoff, Tetrahydrofuran, Acetonitril, Propionitril oder Aceton, ggf. unter Zusatz von Wasser. Besonders vorteilhaft ist wäßriges Aceton (1 bis 20% Wasser), Dioxan/Wasser-Gemische und Tetrahydrofuran/Wasser-Gemische. Die Umsetzung erfolgt bei Temperaturen von Raumtemperatur bis Rückfluß des Lösungsmittels, bevorzugt 30 bis 70°C. Als Säuren eignen sich Mineralsäuren, wie wäßrige Salzsäure, Schwefelsäure oder Phosphorsäure, saure Ionenaustauscher wie Amberlyst 15 oder Dowex 50W x 8.

Im Falle der Verbindungen der Formel XV läßt sich die Oximgruppe -CH=NOH anschließend in die entsprechenden Aldehyde (-CHO) bzw. auch in die entsprechenden Nitrile (-CN) überführen. Bei diesen Verbindungen handelt es sich um wichtige Synthesebausteine zur Herstellung von Wirkstoffen der Formel I (vgl. WO 98/31681).

Bei dem in Verfahrensschritt d) des Verfahrens verwendeten Schritt der Thioalkylierung handelt es sich um ein neues und vorteilhaftes Verfahren zur Umwandlung von Anilinderivaten in Thioetherderivate (Thioalkylierung von Anilinderivaten). Grundsätzlich eignet sich das Verfahren allgemein zur Herstellung von Thioethern der Formel XIX 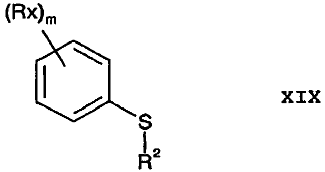 wobei Rx einen beliebigen inerter Rest, m eine Zahl von 0-5 und R² eine C₁-C₆-Alkylgruppe darstellt, dadurch gekennzeichnet, daß man ein Anilin der allgemeinen Formel XX 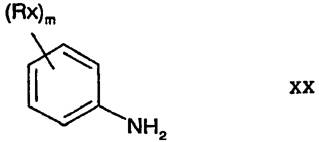 mit einem Dialkyldisulfid der allgemeinen Formel VII

R² - S - S - R² VII

in Gegenwart eines Katalysators umsetzt. Als Katalysator verwendet man bevorzugt Kupferpulver, insbesondere Kupferpulver mit einer Korngröße von weniger als 70 µm, oder elementares Kupfer in anderer Form, wie z.B. Späne, Draht, Granulat, Schrot oder Stäbe.

Bei den Verbindungen der Formel XIX und XX bedeutet Rx einen beliebigen Rest, der unter den gewählten Reaktionsbedingungen während der Umsetzung mit Verbindungen der Formel VII chemisch inert ist. In diesem Sinne kommen für Rx beispielsweise die folgenden Gruppen in Frage: Wasserstoff, Alkyl, Haloalkyl, Halogen, Cyano, Nitro, Alkoxy, Halogenalkoxy, Alkylthio oder heterocyclischen Rest, wie eingangs in der Definition von R⁶ angegeben. Ein heterocyclischer Rest ist insbesondere ein unsubstituierter oder durch Alkyl substituierter 5-gliedrigen heterocyclischer gesättigter, teilgesättigter oder aromatischer Ring aus der Gruppe der Isoxazoline, Isoxazole, Thiazoline, Thiazole, Oxazole, Pyrazole steht. Die Verbindungen der Formel XIX und XX können einen oder mehrere, bevorzugt einen, zwei oder drei Substituenten Rx tragen, die gleich oder verschieden sein können.

Rx bedeutet bevorzugt eine C₁-C₆-Alkylgruppe, z.B. Methyl, Ethyl, Propyl. m bedeutet bevorzugt die Zahl 1 oder 2. Falls m die Zahl 1 bedeutet, steht Rx bevorzugt ortho- oder meta-ständig zur Gruppe -S-R² (im Falle der Verbindungen XIX) bzw. zur Aminogruppe (im Falle der Verbindungen XX). Falls m die Zahl 2 bedeutet, steht der zweite Rest Rx bevorzugt in ortho- und meta-Stellung zur Gruppe -S-R² bzw. zur Aminogruppe.

Thioether der Formel XIX sind wertvolle Zwischenprodukte zur Herstellung von Wirkstoffen in der chemischen Industrie, beispielsweise zur Herstellung von Pflanzenschutzmitteln (z. B. WO 96/11906; WO 98/31676) oder zur Herstellung von Arzneimitteln. Ein häufig verwendetes Verfahren zur Einführung von Alkylthiofunktionen ist der Austausch eines Halogens (EP 0 711 754). Der Nachteil des dort beschriebenen Verfahrens liegt jedoch darin, daß es auf Aromaten beschränkt ist, die durch stark elektronenziehende Reste substituiert sind. Außerdem sind häufig bei der Herstellung hohe Temperaturen erforderlich. Unter diesen Reaktionsbedingungen werden weitere empfindliche funktionelle Gruppen chemisch umgewandelt, so daß komplexe Reaktionsgemische resultieren, deren Aufreinigung umständlich und kostenaufwendig ist bzw. unter Umständen eine Abtrennung der Verunreinigungen überhaupt nicht mehr möglich ist. Hinzu kommt, daß geeignete Vorprodukte auch nicht immer kommerziell erhältlich sind.

Es sind Methoden zur Herstellung von Arylalkylsulfiden aus Anilinen bekannt, die aber schwerwiegende Nachteile aufweisen. Beispielsweise erfordert die Sandmeyer-Reaktion die Verwendung von equimolaren Mengen Kupferalkylthiolat (Baleja, Synth. Commun. 1984, 14: 215-218). Die erzielten Ausbeuten liegen typischerweise lediglich im Bereich von 20 bis 60%.

Eine weitere beschriebene Methode ist die Umsetzung von aromatischen Aminen mit Alkylnitriten in überschüssigem Dialkylsulfid (Giam et.al.J. Chem.Soc. Chem. Commun. 1980, 756-757). Ein Problem hierbei ist, daß teilweise in erheblichem Ausmaß Nebenreaktionen auftreten, die zu schlechten Ausbeuten und hohen Aufwand bei der Produktreinigung führen. Außerdem wurde bei der Durchführung der Reaktion in einem inerten Verdünnungsmittel beobachtet, daß nach einer Induktionsphase eine sehr heftige und schwer zu kontrollierende Reaktion einsetzte, was eine technische Anwendung ausschließt. Es bestand die Aufgabe, ein alternatives Herstellungsverfahren von Thioethern zu finden. Mit Hilfe des erfindungsgemäßen Herstellungsverfahrens können vorteilhaft aromatische Alkylthioether aus Anilinen hergestellt werden. Das Verfahren ermöglicht eine einfache, kostengünstige und effiziente Herstellung unter Berücksichtigung von ökologischen und wirtschaftlich vorteilhaften Gesichtspunkten.

Die Umsetzung des Anilins erfolgt mit einem Dialkyldisulfid und einem organischen Nitrit R-ONO nach dem oben angegebenen Reaktionsschema in Gegenwart eines Katalysators, vorzugsweise elementares Kupfer. Vergleichsversuche zeigen, daß unter den erfindungsgemäßen Bedingungen deutlich bessere Ausbeute erzielt und weniger Nebenprodukte gebildet werden als ohne Katalysator. Die Reaktion ist außerdem gut kontrollierbar und technisch anwendbar.

Die Umsetzung erfolgt nach den im folgenden näher angegebenen Reaktionsbedingungen: Als Lösungsmittel eignen sich Halogenalkane, wie 1,2-Dichlorethan oder Methylenchlorid, Aromaten wie Benzol, Toluol, Chorbenzol, Nitrobenzol. Alternativ kann auch das Dialkyldisulfids selbst im Überschuß als Lösungsmittel eingesetzt werden. Diese Variante ist besonders vorteilhaft. Die Temperaturen für die Umsetzung betragen 40°C bis 150°C, bevorzugt 60 bis 100 °C und insbesondere 70 bis 90 °C. Bei der Umsetzung wird vorteilhaft als Reagenz ein C₁-C₆-Alkylnitrit hinzugefügt. In dieser Hinsicht kommt beispielsweise n-Butylnitrit, (iso)Amylnitrit oder tert-Butylnitrit in Frage. Die Stöchiometrie beträgt in diesem Fall z.B. 1 - 3 Equivalente Alkylnitrit, bevorzugt 1 - 1,5 Equivalente Alkylnitrit. Als Katalysator eignet sich Kupferpulver oder elementares Kupfer in anderer Form, Kupfer-I-Salze, z.B. Kupfer-I-Chlorid, Kupfer-I-Bromid oder Kupfer-I-Jodid, Kupfer-II-Salze,oder elementares Jod, bevorzugt Kupferpulver oder elementares Kupfer in anderer Form. Die Umsetzung erfolgt beispielsweise unter folgenden stöchiometrischen Verhälntissen: Bei Durchführung in einem Lösungsmittel: 1-3 Equivalente Dialkyldisulfid, bevorzugt 1-2 Equivalente. Bei Durchführung ohne zusätzliches Lösungsmittel, d.h. Verwendung des Dialkyldisulfids als Lösungsmittel: Einsatz des Dialkyldisulfids oder Mischungen von Dialkyldisulfiden im Überschuß, wobei diese anschließend destillativ zurückgewonnen werden können Die Produktreinigung erfolgt beispielsweise über Destillation oder Kristallisation (z.B.aus Diisopropylether).

Gegenstand der vorliegenden Erfindung ist ferner ein Verfahren zur Herstellung von Verbindungen X unter Verwendung des zuvor beschriebenen Verfahrens zur Oximierung von substituierten Toluolen XVI (vgl. Verfahrensschrittes a)). In dem folgenden Reaktionsschema 4 ist ein geeignetes Herstellungsverfahren am Beispiel der einer Verbindung X mit R¹=CH₃, R²=CH₃, R³=R4=R⁵=H beschrieben. Grundsätzlich eignet sich das Verfahren auch zur Herstellung von Verbindungen X mit den eingangs definierten Bedeutungen der Reste R¹-R⁵.

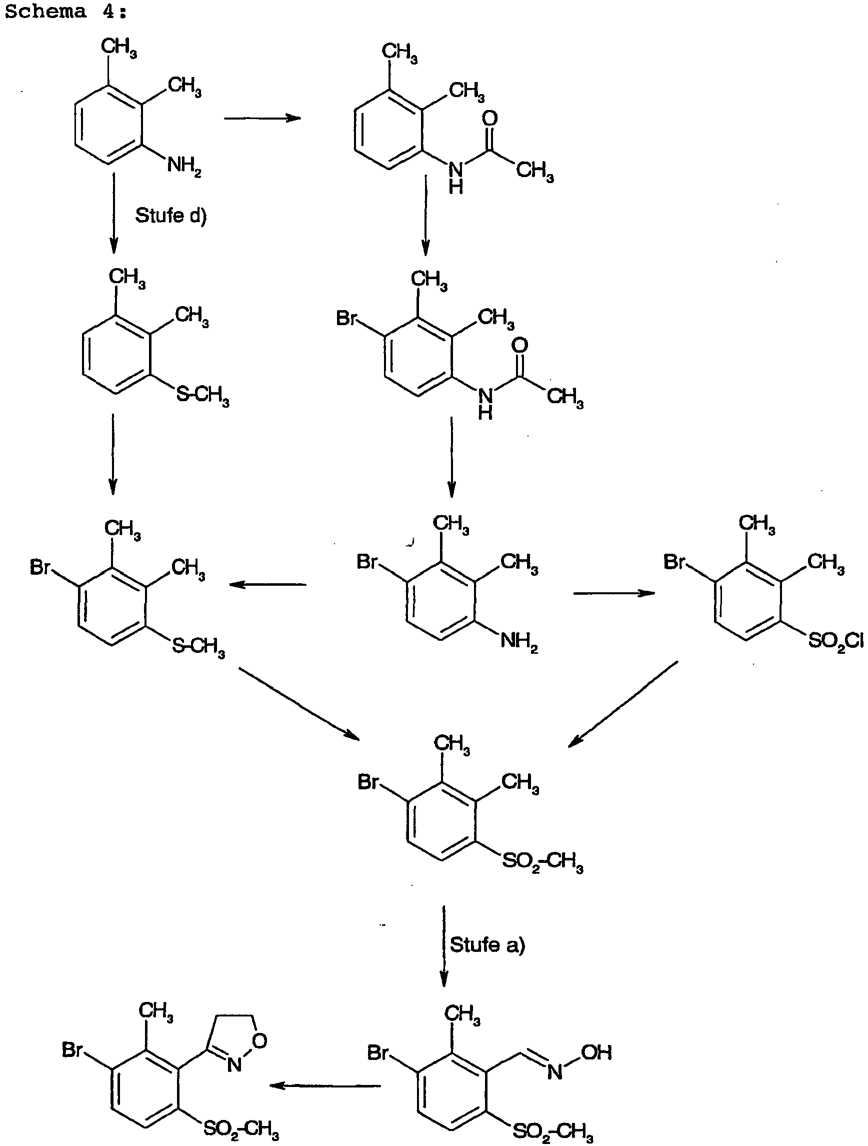

In den folgenden Ausführungsbeispielen wird die Erfindung näher erläutert. Die Beispiele 1 - 9 betreffen die Verfahrensstufen a) - g). Die Beispiele 10 - 26 betreffen die Herstellung von Ausgangs- oder Zwischenverbindungen bzw. enthalten entsprechende Vergleichsbeispiele. Beispiel 27 betrifft die in Schema 4 dargestellte Reaktionsabfolge zur Herstellung von Verbindungen x. Im Folgenden sind nur jene Beispiele erfindungsgemäss, welche unter die Ansprüche fallen.

### Beispiel 1

### Herstellung von 2-Methyl-6-nitro-benzaldoxim

### (Verfahrensstufe a) - Variante A)

Eine Lösung aus 274 g (2,6 mol) n-Butylnitrit (97%ig) und 300 g (2,0 mol) 3-Nitro-o-xylol (97%ig) in 750 ml Dimethylformamid wird auf -55-60°C gekühlt und bei dieser Temperatur eine Lösung von 522 g (4,56 mol) Kalium-tert.-butylat in 750 ml Dimethylformamid innerhalb von 2,5 Stunden zugetropft. Die Farbe der Lösung ändert sich dabei von gelb nach tiefrot und die Konsistenz wird zähflüssig. Die Reaktion wird per HPLC verfolgt. Zur Aufarbeitung werden zunächst 300 ml Wasser zugesetzt und anschließend ca. 300 ml Eisessig bis der pH-Wert 5-6 erreicht ist. Die Temperatur steigt dabei auf -10°C an und es bildet sich eine gelbe Suspension. Der Ansatz wird anschließend auf 6 kg Eiswasser gegossen und der gebildete Rückstand abgesaugt, mit 5 1 Wasser nachgewaschen und im Trockenschrank bei 30°C über Nacht getrocknet.

Man erhält 339 g eines hellbeigen Rohprodukts, das durch Suspendieren in ca. 3 1 Toluol bei 80-90 °C für 2 Stunden von den Verunreinigungen befreit wird. Nach dem Abkühlen wird das Produkt abgesaugt und getrocknet. Man erhält 276g 2-Nitro-6-methyl-benzaldoxim.

Ausbeute: 77% Fp.: 190-192°C Reinheit (nach HPLC): 98%.

### Beispiel 2:

### Herstellung von 2-Methyl-6-nitro-benzaldoxim

### (Verfahrensstufe a) - Variante B)

1200 ml wasserfreies DMF werden in einem 41-Reaktionskolben vorgelegt und auf - 40°C gekühlt. Unter Rühren werden bei dieser Temperatur 336,5 g (4,56 mol) Kalium-methylat (95 %) zugegeben und suspendiert. Anschließend wird ein Gemisch aus 300 g (1,92 mol) 3-Nitro-o-xylol (97 %) und 274 g (2,52 mol) n-Butylnitrit (95 %) über 7 Stunden bei - 40°C zugetropft (bei entsprechender Kühlleistung kann diese Zugabedauer beliebig verkürzt werden; eine Verlängerung wurde noch nicht geprüft; Temperaturschwankungen zwischen - 35 und - 45°C sind tolerabel). Der vollständige Umsatz des Ausgangsmaterials wird über HPLC überprüft. Anschlie-ßend wird der Reaktionsaustrag in eine Mischung aus 300 ml Wasser und 300 ml Eisessig bei - 5 bis 0°C unter Rühren gegeben. Der Ansatz wird dann auf 6 kg Eiswasser gegeben, der Feststoff durch Filtration abgetrennt (unproblematisch, Filterwiderstand noch nicht bestimmt) und 2 mal mit je 500 ml Wasser gewaschen (Achtung: Rohprodukt stark riechend). Die Reinigung des Rohproduktes (HPLC: 96 Fl.-%) erfolgt durch Suspendieren des feuchten Feststoffes in 800 ml Toluol über 1,5 h. Der Feststoff wird abfiltriert (unproblematisch, Filterwiderstand noch nicht bestimmt) und im Vakuumtrockenschrank bei 50°C getrocknet.

Ausbeute: 306 g (HPLC: 99,4 Fl.-% Produkt; E/Z-Mischung), entsprechend 85 % d. Th.

### Beispiel 3

### Herstellung von 3-(2-Methyl-6-nitrophenyl)-4,5-dihydroisoxazol

### (Verfahrensstufe b))

a) Zu einer Lösung von 5 g (28 mmol) 2-Methyl-6-nitro-benzaldoxim in 50 ml Acetonitril wird bei 60°C eine kleine Menge einer Lösung von 3,71 g (28 mmol) N-Chlorsuccinimid in 30 ml Acetonitril gegeben. Nachdem die Reaktion angesprungen ist, wird bei 40-50°C die restliche Lösung langsam zugetropft. Der Ansatz wird noch 20 Minuten nachgerührt bis nach HPLC die Umsetzung vollständig ist. Es ergibt sich eine orangefarbene Lösung, die vorsichtig eingeengt wird. Der Rückstand wird ca. 1,5 Stunden in 50 ml Toluol supendiert und die Lösung vom Succinimid abgetrennt. Das Filtrat hat immer noch eine orangerote Färbung. Die Lösung wird in einen Miniautoklaven gefüllt und 30 bar Ethylen aufgepreßt. Dann wird innerhalb von 5 Stunden eine Lösung von 4,7 g Natriumhydrogencarbonat in 50 ml Wasser zudosiert und noch 5 Stunden bei 30 bar Ethylendruck nachgerührt. Zur Aufarbeitung werden die Phasen getrennt, die Toluolphase noch 2x mit NaHCO₃-Lösung und lx mit Wasser gewaschen, getrocknet und eingeengt. Ausbeute: 4,9 g (86%). bräunliche Kristalle, Smp.: 100-105°C.
   1H-NMR (CDC1₃): δ = 8.00 (d, 1H) ; 7.57 (d, 1H); 7.49 (t, 1H); 4.60 (t, 2H); 3.32 (t, 2H); 2.41 (s, 3H).
b) 100 g 2-Methyl-6-nitro-benzaldoxim werden in 750 ml Eisessig gelöst, wonach 2 Stunden Chlor eingeleitet wird. Überschüssiges Chlor wird mit Stickstoff ausgegast . Anschließend wird der Eisessig abdestilliert und der Rückstand in 1000 ml Toluol suspendiert. Das Reaktionsgemisch wird in den Autoklaven gefüllt und 6 bar Ethylen wird aufgepresst. Innerhalb einer Stunde werden 55,6 g Triethylamin (1 Äq.) in 300 ml Toluol zudosiert und 10 h bei Raumtemperatur und 6 bar Ethylen nachgerührt. Der Ansatz wird 1 Mal mit gesättigter wässriger NaHCO₃-Lösung und 1 Mal mit Wasser gewaschen. Die organische Phase wird über Natriumsulfat getrocknet, abfiltriert und einrotiert. Ausbeute: 96,3g (87% d. Th.).

### Beispiel 4

### Herstellung von 2-(4,5-Dihydroisoxazol-3-yl)-3-methylanilin

### (Verfahrensstufe c))

a) In einen Hydrierautoklaven gibt man eine Lösung von 117 g (0,57 mol) 3-(2-Methyl-6-nitrophenyl)-4,5-dihydroisoxazol in 1,2 l Essigsäureethylester und 11,7 g eines Katalysators, der 5 Gew.-% Platin auf Kohle enthält. Dann wird der Autoklav zweimal mit Stickstoff gespült. Anschließend wird bei 20 bar Wasserstoffdruck unter intensiver Rührung 48 Stunden bei 25-30°C hydriert. Der Reaktionsaustrag wird über Kieselgel abgesaugt und das Lösungsmittel i.Vak. abgezogen. Man erhält 94 g eines braunen Feststoffs, der in Methyl-tert.butylether und Wasser aufgenommen und mit 1M Salzsäure extrahiert wird. Die wässrige Phase wird auf pH 10-11 eingestellt und mit . Methylenchlorid extrahiert. Die Methylenchloridphase wird über Magnesiumsulfat getrocknet und das Lösungsmittel abgezogen.
   Ausbeute 87 g (87%) oranger Feststoff, Fp.: 86-88°C, Reinheit nach HPLC 97%.
   Durch Aufrühren mit Methyl-tert.butylether unter Rückfluß kann das Produkt weiter gereinigt werden: Fp.: 90-91°C, Reinheit nach HPLC 100%.
b) In einen Hydrierautoklaven gibt man eine Lösung von 1000 g (4,85 mol) 3-(2-Methyl-6-nitrophenyl)-4,5-dihydroisoxazol in 5,5 l Methanol und 4,6 g eines Katalysators, der 10 Gew.-% Pd auf Kohle enthält. Dann wird der Autoklav zweimal mit Stickstoff gespült. Anschließend wird bei 2,5 bar Wasserstoffdruck unter intensiver Rührung 17 Stunden bei 25-30 °C hydriert. Der Reaktionsaustrag wird über Kieselgel abgesaugt und das Lösungsmittel i.Vak. abdestilliert.
   Man erhält 781,7 g eines hellbraunen Feststoffs.
   Ausbeute 781,7 g (85%) (Gehalt nach HPLC 93%).

### Beispiel 5

### Herstellung von 3-(2-Methyl-6-methylthiophenyl)-4,5-dihydroisoxazol

### (Verfahrensstufe d))

19,5 g (170 mmol) tert. Butylnitrit und 20 g Kupferpulver werden in 30 ml Dimethyldisulfid vorgelegt und bei 50 bis 55°C eine Lösung von 20 g (114 mmol) 2-(4,5-dihydroisoxazol-3-yl)-3-methylanilin in 100 ml Dimethyldisulfid zugetropft. Anschließend wird 1,5 Stunden bei 60 °C nachgerührt. Zur Aufarbeitung wird vom Feststoff abgesaugt, mit Methylenchlorid verdünnt und mit verdünnter Salzsäure extrahiert. Die organische Phase wird mit gesättigter wässriger NaHCO₃-Lösung gewaschen, über Natriumsulfat getrocknet, abfiltriert und eingeengt. Überschüssiges Dimethyldisulfid wird im Ölpumpenvakuum entfernt.

Man erhält 23,4 g (99%) eines dunklen Öls, das nach einiger Zeit erstarrt. (Gehalt nach HPLC 100 %). Das Produkt kann durch Aufrühren in Methyl-tert.butylether weiter gereinigt werden. Fp.: 66-67°C

### Beispiel 6

### Herstellung von 3-(3-Brom-2-methyl-6-methylthiophenyl)-4,5-dihydroisoxazol

### (Verfahrensstufe e))

Zu 120 ml conc. Schwefelsäure werden bei 0°C portionsweise 10 g (48 mmol) 3-(2-Methyl-6-methylthiophenyl)-4,5-dihydroisoxazol gegeben und ca. 30 Minuten gerührt. Dann werden 3,7 g (23 mmol) Brom zugetropft und 2,5 Stunden bei 0°C gerührt. Anschließend läßt man das Gemisch in ca. 45 Minuten auf Raumtemperatur erwärmen. Dabei bildet sich eine homogene Lösung. Zur Aufarbeitung wird der Reaktionsansatz auf Eiswasser gegossen und dreimal mit Methylenchlorid extrahiert. Die organische Phase wird mit Natriumhydrogencarbonatlösung gewaschen, mit Magnesiumsulfat getrocknet und eingeengt. Man erhält 11,4 g Rohprodukt, das ohne weitere Reinigung in die nächste Stufe eingesetzt wird.

### Beispiel 7

### Herstellung von 3-(3-Brom-2-methyl-6-methylsulfonylphenyl)-4,5-dihydroisoxazol

### (Verfahrensstufe f))

Zu einer Lösung von 11,4 g (40 mmol) 3-(3-Brom-2-methyl-6-methylthiophenyl)-4,5-dihydroisoxazol und 400 mg Natriumwolframat-Hydrat in 100 ml Eisessig werden bei max. 45°C 11,3 g (100 mmol) 30 %iges Wasserstoffperoxid getropft. Der Ansatz wird über Nacht bei Raumtemperatur gerührt. Zur Aufarbeitung wird auf Eiswasser gegossen, mit Methylenchlorid extrahiert, die organische Phase mit wässriger Natriumsulfitlösung gewaschen, über Magnesiumsulfat getrocknet und eingeengt. Ausbeute: 9,6 g. Zur Reinigung kann das Produkt aus 65 ml i-Propanol umkristallisiert werden.

Ausbeute: 7,7 g (50 % über 2 Stufen) Fp.: 137-139°C.

### Beispiel 8

### 1-Methyl-4-(3-(4,5-dihydroisoxazol-3-yl)-2-methyl-4-methylsulfonyl-benzoyl)-5-hydroxypyrazol

### (Verfahrensstufe g) - Variante A)

In einen 3,5 1-Autoklaven wurden 2,2 1 1,4-Dioxan, 100 g (0,315 Mol) 3-(3-Brom-2-methyl-6-methylsulfonylphenyl)-4,5-dihydroisoxazol, 30,82 g (0,315 Mol) 1-Methyl-5-hydroxypyrazol, 87 g (0,63 Mol) Kaliumcarbonat, 63,5 g (0,63 Mol) Triethylamin und 11,2 g (0,016 Mol) Bistriphenylphosphin-Palladiumdichlorid gegeben. Anschließend wurde zweimal mit Stickstoff gespült, 10 kg/cm² Kohlenmonoxyd aufgepreßt und unter Rühren auf 130°C erhitzt. Dann wurde der Kohlenmonoxyd-Druck auf 20 kg/cm² erhöht und 24 h bei 130°C gerührt. Nun wurde im Vakuum eingeengt und der Rückstand in Wasser aufgenommen. Die wässrige Phase mit einem pH-Wert von 11 wird mit Dichlormethan extrahiert. Die organische Phase wird verworfen. Die wäßrige Phase wird mit 18%iger Salzsäure auf pH 4 eingestellt. Der ausgefallene Niederschlag wurde abfiltriert, dreimal mit Wasser gewaschen und im Vakuum bei 40°C getrocknet. Man erhält 85 g Produkt. Das Filtrat wird mit Dichlormethan extrahiert. Nach Trocknung der organischen Phase mit Natriumsulfat und Abziehen des Lösungsmittels erhält man weitere 12,7 g Produkt.

Ausbeute 97,7 g (85,6 %). Schmp.: 215-219 °C, ¹H-NMR (CDC1₃): δ = 2,38 (s); 3,23 (s); 3,41 (bs); 3,74 (s); 4,61 (t); 7,37 (s); 7,64 (d); 8,16 (d).

### Beispiel 9

### 1-Methyl-4-(3-(4,5-dihydroisoxazol-3-yl)-2-methyl-4-methylsulfonyl-benzoyl)-5-hydroxypyrazol

### (Verfahrensstufe g) - Variante B)

In einen 3,5 1-Autoklaven wurden 2 l 1,4-Dioxan, 250 g (0,77 Mol) 3-(3-Brom-2-methyl-6-methylsulfonylphenyl)-4,5-dihydroisoxazol, 77 g (0,77 Mol) 1-Methyl-5-hydroxypyrazol, 269 g (1,93 Mol) Kaliumcarbonat, 197 g (1,93 Mol) Triethylamin, 1,39 g (0,0077 Mol) Palladium(II)-chlorid und 4,12 g (0,0154 Mol) Triphenylphosphin gegeben. Anschließend wurde zweimal mit Stickstoff gespült, unter Rühren auf 130°C erhitzt und bis aus einen Druck von 6 kg/cm² Kohlenmonoxid aufgepreßt. Durch kontinuierliche Zugabe von Kohlenmonoxid wurde der Kohlenmonoxyd-Druck konstant auf 6 kg/cm² gehalten und 36 h bei 130°C gerührt. Nun wurde mit 1 l VE-Wasser versetzt, das ausgefallene Palladium mit einem Blaubandfilter (Porengröße 2 bis 3 µ) abfiltiert und mit Wasser gewaschen. Anschließend wurden Dioxan, Triethylamin und ein Teil des Wassers einstufig abdestilliert. (150 mbar oder Normaldruck). Die wäßrige Phase wurde mit 20%iger Schwefelsäure auf pH 2,5 eingestellt und unter Nachjustierung des pH-Wertes über 12 h bei 5 °C nachgerührt. Der ausgefallene Niederschlag wurde abfiltriert, dreimal mit Wasser gewaschen und im Vakuum bei 70°C getrocknet. Man erhält 227 g Produkt (ber. 100 %).

Ausbeute 227 g (81 %). Schmp.: 215-219 °C, ¹H-NMR (CDC1₃): δ = 2,38 (s); 3,23 (s); 3,41 (bs); 3,74 (s); 4,61 (t); 7,37 (s); 7,64 (d); 8,16 (d).

Palladiumrückgewinnungsrate auf Filter: 85 - 98 %

Elementaranalyse abfiltriertes Palladium (getrocknet): Pd 48 %, O 22 %, C 11 %, H 1,3 %, P 0,2 %, S 0,2 %, Br < 0,5 %, Cl < 0,5 %, N < 0,5 %.

### Beispiel 10

### Herstellung von 4-Brom-2-(4,5-dihydroisoxazol-3-yl)-3-methylanilin

30 g (170 mmol) 2-(4,5-dihydroisoxazol-3-yl)-3-methylanilin werden in 400 ml Acetonitril gelöst und 94 g (0,68 mol) Kaliumcarbonat zugegeben. Anschließend werden unter kräftigem Rühren portionsweise 84 g (174 mmol) Tetrabutylammoniumtribromid bei Temperaturen < 30°C zugegeben. Zur Aufarbeitung wird vom Feststoff abgesaugt, mit Methylenchlorid verdünnt und mit Wasser extrahiert. Nach dem Abziehen des Lösungsmittels wird der Rückstand wieder in Methyl-tert.butylether aufgenommen und noch zweimal mit Wasser gewaschen. Die organische Phase wird getrocknet und eingeengt.

Ausbeute 20,4 g (47%) braunen Feststoff, Fp.: 126-130°C, Reinheit nach HPLC 97%

### Beispiel 11

### Herstellung von 4-Brom-2-(4,5-dihydroisoxazol-3-yl)-3-methyl-benzolsulfonylchlorid

Eine Lösung von 9 g (35 mmol) 4-Brom-2-(4,5-dihydroisoxazol-3-yl)-3-methylanilin in 50 ml Eisessig wird bei 15°C zu 15 ml conc. Salzsäure gegeben. Dann wird bei 5-10°C eine Lösung von 2,44 g (35 mmol) Natriumnitrit in 10 ml Wasser zugetropft und 1 Stunde bei 5°C nachgerührt. Diese Lösung wird dann bei Raumtemperatur in eine Mischung aus einer Lösung von 47 g (0,74 mol) Schwefeldioxid in 100 ml Eisessig und einer Lösung von 2,23 g (13 mmol) Kupfer(II)chlorid in 5 ml Wasser zugetropft. Der Ansatz wird 1 Stunde bei Raumtemperatur nachgerührt, anschließend auf 300 ml Eiswasser gegeben und mit Methylenchlorid extrahiert.. Die organische Phase wird mit Wasser gewaschen, mit Magnesiumsulfat getrocknet und eingeengt.

Ausbeute 11,8 g (99 %), Reinheit nach HPLC 96 %

In den folgenden Ausführungsbeispiele wird die Herstellung von Benzaldoximen der Formel XV (Verfahrensstufe a) näher beschrieben:

### Beispiel 12

### Herstellung von 2-Methyl-6-nitro-benzaldoxim (Variante A)

Eine Lösung aus 274 g (2,6 mol) n-Butylnitrit (97%ig) und 300 g (2,0 mol) 3-Nitro-o-xylol (97%ig) in 750 ml Dimethylformamid wird auf -55 bis -60°C gekühlt und bei dieser Temperatur eine Lösung von 522 g (4,56 mol) Kalium-tert.-butylat in 750 ml Dimethylformamid innerhalb von 2,5 Stunden zugetropft. Die Farbe der Lösung ändert sich dabei von gelb nach tiefrot und die Konsistenz wird zähflüssig. Die Reaktion wird per HPLC verfolgt. Zur Aufarbeitung werden zunächst 300 ml Wasser zugesetzt und anschließend ca. 300 ml Eisessig bis der pH-Wert 5-6 erreicht ist. Die Temperatur steigt dabei auf -10°C an und es bildet sich eine gelbe Suspension. Der Ansatz wird anschließend auf 6 kg Eiswasser gegossen und der gebildete Rückstand abgesaugt, mit 5 l Wasser nachgewaschen und im Trockenschrank bei 30°C über Nacht getrocknet. Man erhält 339 g eines hellbeigen Rohprodukts, das durch Suspendieren in ca. 3 l Toluol bei 80-90°C für 2 Stunden von den Verunreinigungen befreit wird. Nach dem Abkühlen wird das Produkt abgesaugt und getrocknet. Man erhält 276g 2-Nitro-6-methyl-benzaldoxim.

Ausbeute: 77% Fp.: 190-192°C Reinheit (nach HPLC): 98%

### Beispiel 13

### Herstellung von 2-Methyl-6-nitro-benzaldoxim (Variante B)

1200 ml wasserfreies DMF werden in einem 41-Reaktionskolben vorgelegt und auf - 40°C gekühlt. Unter Rühren werden bei dieser Temperatur 336,5 g (4,56 mol) Kaliummethylat (95 %) zugegeben und suspendiert. Anschließend wird ein Gemisch aus 300 g (1,92 mol) 3-Nitro-o-xylol (97 %) und 274 g (2,52 mol) n-Butylnitrit (95 %) über 7 Stunden bei - 40°C zugetropft (bei entsprechender Kühlleistung kann diese Zugabedauer beliebig verkürzt werden). Der vollständige Umsatz des Ausgangsmaterials wird über HPLC überprüft. Anschließend wird der Reaktionsaustrag in eine Mischung aus 300 ml Wasser und 300 ml Eisessig bei - 5 bis 0°C unter Rühren gegeben. Der Ansatz wird dann auf 6 kg Eiswasser gegeben, der Feststoff durch Filtration abgetrennt und 2 mal mit je 500 ml Wasser gewaschen.

Die Reinigung des Rohproduktes (HPLC: 96 Fl.-%) erfolgt durch Suspendieren des feuchten Feststoffes in 800 ml Toluol über 1,5 h. Der Feststoff wird abfiltriert und im Vakuumtrockenschrank bei 50°C getrocknet.

Ausbeute: 306 g (HPLC: 99,4 Fl.-% Produkt; E/Z-Mischung), entsprechend 85 % d. Th.

### Beispiel 14

### Herstellung von 2-Chlor-6-nitro-benzaldoxim

Eine Lösung aus 4,1 g (40 mmol) n-Butylnitrit (97%ig) und 5 g (29 mmol) 2-Chlor-6-nitro-toluol in 50 ml Dimethylformamid wird auf -55 bis -60°C gekühlt und bei dieser Temperatur eine Lösung von 3,3 g (29,5 mmol) Kalium-tert.-butylat in 30 ml Dimethylformamid innerhalb von 20 Minuten zugetropft. Die Reaktion wird per HPLC verfolgt. Zur Aufarbeitung werden zunächst Wasser zugesetzt und anschließend mit Eisessig auf pH 5-6 eingestellt. Das Produkt wird durch Extraktion mit Essigester isoliert. Man erhält 5,7 g 2-Chlor-6-nitro-benzaldoxim. ¹H-NMR (CDC1₃): δ = 8.00 (d, 1H); 7.84 (s, 1H); 7.76 (d, 1H); 7.52 (t, 1H).

### Beispiel 15

### Herstellung von 3-Chlor-2-methyl-6-methylsulfonyl-benzaldoxim

Eine Lösung aus 12,7 g (119 mmol) n-Butylnitrit (97%ig) und 20 g (92 mmol) 2,3-Dimethyl-4- methylsulfonyl-chlorbenzol in 100 ml Dimethylformamid wird auf -55 bis -60°C gekühlt und bei dieser Temperatur eine Lösung von 16,8 g (147 mmol) Kalium-tert.-butylat in 70 ml Dimethylformamid innerhalb von 30 Minuten zugetropft. Die Reaktion wird per HPLC verfolgt. Zur Aufarbeitung werden zunächst 50 ml Wasser zugesetzt und anschließend mit ca. 30 ml Eisessig auf pH 5-6 eingestellt. Der Ansatz wird anschließend auf 0,7 kg Eiswasser gegossen und die wäßrige Phase mit Methylenchlorid extrahiert. Die organische Phase wird mit Natriumhydrogencarbonatlösung gewaschen, über Magnesiumsulfat getrocknet und eingeengt. Man erhält 18,4 g eines hellbeigen Rohprodukts, das durch Umkristallisieren aus ca. 30 ml Toluol gereinigt wird.

Ausbeute: 6,15 g (27 %) weiße Kristalle Fp.: 164-168°C Reinheit (nach HPLC): 100%

### Beispiel 16

### Herstellung von 3-Brom-2-methyl-6-methylsulfonyl-benzaldoxim

Eine Lösung aus 2,1 g (20 mmol) n-Butylnitrit (97%ig) und 4 g (15 mmol) 2,3-Dimethyl-4-methylsulfonyl-brombenzol in 50 ml Dimethylformamid wird auf -55 bis -60°C gekühlt und bei dieser Temperatur eine Lösung von 2,8 g (25 mmol) Kalium-tert.-butylat in 35 ml Dimethylformamid innerhalb von 20 Minuten zugetropft. Die Reaktion wird per HPLC verfolgt. Zur Aufarbeitung werden zunächst 10 ml Wasser zugesetzt und anschließend mit ca. 9 ml Eisessig auf pH 5-6 eingestellt. Der Ansatz wird anschließend auf 100 ml Eiswasser gegossen und die wäßrige Phase mit Methylenchlorid extrahiert. Die organische Phase wird mit Natriumhydrogencarbonatlösung gewaschen, über Magnesiumsulfat getrocknet und eingeengt. Man erhält 3,6 g eines öligen Rohprodukts (nach HPLC 90%ig), das durch Umkristallisieren aus Toluol gereinigt werden kann.

Ausbeute: 1,22 g (27 %) Fp.: 192-194°C Reinheit (nach HPLC): 99 %

### Beispiel 17

### Herstellung von 3-Hydroxyimino-2-methyl-4-methylsulfonyl-benzoesäurediphenylamid

a) Herstellung des Vorprodukts 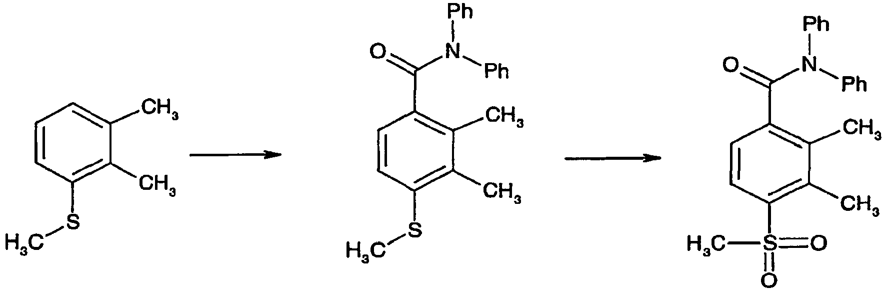 5 g (3 mmol) 2,3 - Dimethylthioanisol und 7,6 g (33 mmol) Diphenylcarbamoylchlorid werden in 50 ml 1,2-Dichlorethan gelöst und bei Raumtemperatur mit 4,8 g (36 mmol) wasserfreiem Aluminiumchlorid versetzt. Das Reaktionsgemisch wird 3 Stunden am Rückfluß gekocht, auf eine Mischung aus Eis und konzentrierter Salzsäure gegossen und die wäßrige Phase zweimal mit Methylenchlorid extrahiert. Die organische Phase wird mit Natriumhydrogencarbonatlösung gewaschen, über Magnesiumsulfat getrocknet und eingeengt. Man erhält 10,8 g Rohprodukt, das durch Kieselgelchromatographie mit Toluol/Essigester als Eluent gereinigt wird. Ausbeute 7,8 g 2,3-Dimethyl-4-methylthio-benzoesäurediphenylamid.
   Zu einer Lösung von 7 g (20 mmol) 2,3-Dimethyl-4-methylthio-benzoesäurediphenylamid und 200 mg Natriumwolframat-Hydrat in 50 ml Eisessig werden bei max. 45 °C 5,7 g (50 mmol) 30 %iges Wasserstoffperoxid getropft. Der Ansatz wird über Nacht bei Raumtemperatur gerührt. Zur Aufarbeitung wird auf Eiswasser gegossen, mit Methylenchlorid extrahiert, die organische Phase mit wässriger Natriumsulfitlösung gewaschen, über Magnesiumsulfat getrocknet und eingeengt.
   Ausbeute: 7,4 g 2,3-Dimethyl-4-methylsulfonyl-benzoesäuredi-phenylamid, Fp.: 155-165°C
b) Herstellung von 3-Hydroxyimino-2-methyl-4-methylsulfonyl-benzoesäurediphenylamid
   Eine Lösung aus 0,7 g (6,9 mmol) n-Butylnitrit (97%ig) und 2 g (5,3 mmol) 2,3-Dimethyl-4-methylsulfonyl-benzoesäuredi-phenylamid in 30 ml Dimethylformamid wird auf -55 bis -60°C gekühlt und bei dieser Temperatur eine Lösung von 1,4 g (12 mmol) Kalium-tert.-butylat in 10 ml Dimethylformamid innerhalb von 20 Minuten zugetropft. Die Reaktion wird per HPLC verfolgt. Zur Aufarbeitung werden zunächst 10 ml Wasser zugesetzt und anschließend mit Eisessig auf pH 5-6 eingestellt. Der Ansatz wird anschließend auf 100 ml Eiswasser gegossen und die wäßrige Phase mit Essigsäureethylester extrahiert. Die organische Phase wird mit Natriumhydrogencarbonatlösung gewaschen, über Magnesiumsulfat getrocknet und eingeengt. Man erhält 3,0 g eines teilweise kristallinen Rohprodukts das durch Kieselgelchromatographie mit Toluol/Aceton als Eluent gereinigt wird.
   Ausbeute: 1,0 g (46 %) Fp.: 208-211°C.

### Beispiel 18

### Herstellung von 3-Brom-2-methyl-6-methylsulfonyl-benzaldehyd

7,1 g 3-Brom-2-methyl-6-methylsulfonyl-benzaldoxim (23mmol) werden in einer Mischung aus 17 g 5%iger Salzsäure, 2 g 37%iger Formaldehydlösung, 15ml Wasser und 30 ml Tetrahydrofuran 32 Stunden bei 65 °C gerührt. Dabei werden in Portionen von 0,5 g weitere 3,5 g 37%ige Formaldehydlösung zugegeben. Anschließend wird auf Raumtemperatur abgekühlt und das Produkt abgesaugt.

Man erhält 5,1 g (79 %) Reinheit 94 % (nach GC).

### Beispiel 19

### Herstellung von 2-Methyl-6-nitro-benzaldehyd

14 g 2-Methyl-6-nitro-benzaldoxim (80mmol) werden in einer Mischung aus 55 ml 5%iger Salzsäure, 37 g 37%iger Formaldehydlösung 50 ml Wasser und 100 ml Tetrahydrofuran 24 Stunden bei 65°C gerührt. Anschließend werden die Phasen getrennt und die dunkle Phase mit Methylenchloid/wasser extrahiert. Die organische Phase wird mit Natriumsulfat getrocknet und eingeengt. Man erhält 10,1 g Rohprodukt das durch Filtration über Kieselgel mit Toluol als Laufmittel gereinigt wird.

Ausbeute: 7,2 g (54 %)

### Beispiel 20

### Herstellung von 2-Methyl-6-nitro-benzonitril

Eine Lösung aus 16 g (150 mmol) n-Butylnitrit (97%ig) und 7,7 g (50 mmol) 3-Nitro-o-xylol (97%ig) in 50 ml Dimethylformamid wird auf -5 bis -10°C gekühlt und bei dieser Temperatur eine Lösung von 11 g (100 mmol) Kalium-tert.-butylat in 50 ml Dimethylformamid innerhalb von 1,5 Stunden zugetropft. Das Reaktionsgemisch wird noch 6 Tage bei Raumtemperatur gerührt. Zur Aufarbeitung wird auf Eiswasser gegossen, mit Salzsäure auf einen pH-Wert von 1 eingestellt und die wäßrige Phase mit Essigsäureethylester extrahiert. Die organische Phase wird mit Wasser gewaschen, über Magnesiumsulfat getrocknet und eingeengt. Man erhält 8,2 g Produkt.Durch Kieselgelchromatographie mit Toluol als Eluent kann das 2-Methyl-6-nitro-benzonitril gereinigt werden. Fp.: 101-103°C.

In den folgenden Ausführungsbeispiele wird die Herstellung von Thiothern der Formel VIIIa (Verfahrensstufe d) näher beschrieben:

### Beispiel 21

a) Vergleichsbeispiel
   Bei der Umsetzung von 2,3-Dimethylanilin mit Dimethyldisulfid und tert.Butylnitrit in Methylenchorid als Lösungsmittel erhält man das gewünschte Produkt C nur zum kleinen Teil. Als Hauptprodukte nach GC-Analyse konnten die Dimerisierungsprodukte A und B identifiziert werden. Auch bei der Umsetzung in überschüssigem Dimethyldisulfid entsteht das Dimer A. 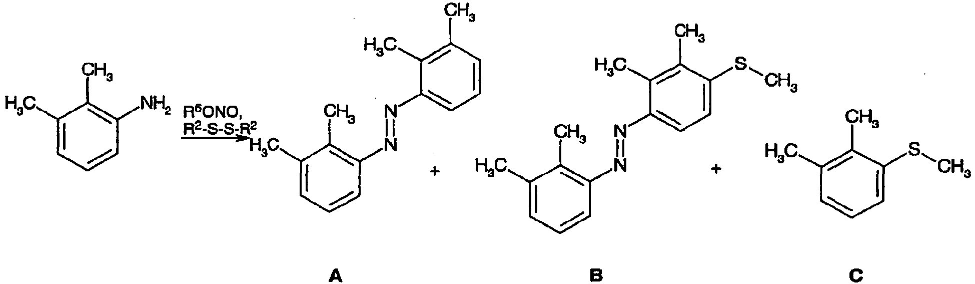
b) Erfindungsgemäßes Verfahren
   Analog zu der in a) beschriebenen Methode erfolgt die Umsetzung von 2,3-Dimethylanilin mit Dimethyldisulfid und tert.Butylnitrit in Methylenchorid als Lösungsmittel, wobei zusätzlich Cu-Pulver als Katalysator hinzugefügt wird. Die Umsetzung erfolgt einheitlich zum gewünschten Dimethylthioanisol C. Die Dimerisierungsprodukte A und B konnten durch GC-Analyse nicht identifiziert werden.

### Beispiel 22

a) Vergleichsbeispiel
   Bei der Umsetzung von 2-(4,5-dihydroisoxazol-3-yl)-3-methylanilin mit Dimethyldisulfid und tert.Butylnitrit ohne Katalysator bilden sich Nebenprodukte. Man erhält ein Gemisch aus A und B im Verhältnis 2:1 nach HPLC-Flächenprozent. 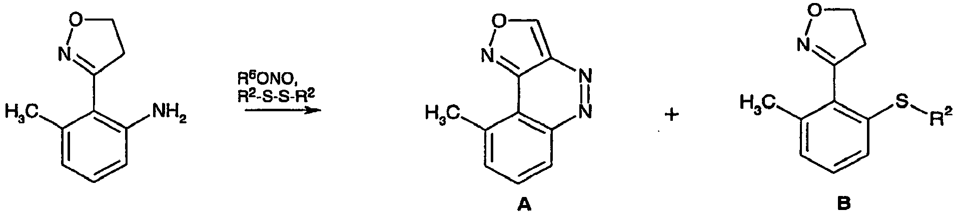
b) Erfindungsgemäßes Verfahren
   Analog zu der in a) beschriebenen Methode wird die Umsetzung in Gegenwart von Cu-Pulver durchgeführt. Das Nebenprodukt A ist in diesem Fall nicht nachweisbar.

### Beispiel 23

### Herstellung von 2,3-Dimethylthioanisol

a) 355 g (3,44 mol) tert. Butylnitrit und 250 g Kupferpulver (3,9 mol) werden in 1250 ml Dimethyldisulfid vorgelegt und bei 50 bis 52°C eine Lösung von 250g (2,07 mol) 2,3-Dimethylanilin in 1000 ml Dimethyldisulfid zugetropft. Anschließend wird 1,5 Stunden bei 75°C bis 80°C nachgerührt. Zur Aufarbeitung wird abgekühlt, durch Kieselgur abgesaugt und das Filtrat mit gesättigter wässriger NaHCO₃-Lösung gewaschen. Zur Reinigung des Produkts wird die organische Phase destillativ getrennt. Zunächst wird bei Normaldruck das überschüssige Dimethyldisulfid abgetrennt. Man gewinnt 1446 g Dimethyldisulfid (Reinheit >97 % nach GC) zurück. Anschließend wird i.Vak.(0,1 mbar) fraktioniert destilliert.
   Ausbeute: 261,3 g (83 %), Reinheit nach GC 97,5 %
b) 14,2 g (124 mmol) tert. Butylnitrit und 2,5 g (40 mmol) Kupferpulver werden in 50 ml Dimethyldisulfid vorgelegt und bei 50 bis 52°C eine Lösung von 10g (81 mmol) 2,3-Dimethylanilin in 50 ml Dimethyldisulfid zugetropft. Anschließend wird 1,5 Stunden bei 75 bis 80°C nachgerührt. Nach GC-Analyse hat sich das Anilin zu 100% in das gewünschte 2,3-Dimethylthioanisol umgesetzt.

### Beispiel 24

### Herstellung von 2-Methyl-6-nitrothioanisol

226 g (1,97 mol) tert. Butylnitrit und 100 g Kupferpulver werden in 300 ml Dimethyldisulfid vorgelegt und bei 50 bis 55°C eine Lösung von 200g (1,32 mol) 2-Methyl-6-nitroanilin in 700 ml Dimethyldisulfid zugetropft. Anschließend wird 8 Stunden bei 75°C nachgerührt. Zur Aufarbeitung wird vom Feststoff abgesaugt, mit Methylenchlorid verdünnt und mit verdünnter Salzsäure extrahiert. Die organische Phase wird mit gesättigter wässriger NaHCO₃-Lösung gewaschen, über Natriumsulfat getrocknet, abfiltriert und einrotiert. Überschüssiges Dimethyldisulfid wird am Ölpumpenvakuum entfernt. Man erhält 271 g (99 %) eines dunkelroten Öls, Reinheit nach HPLC 87 %.

### Beispiel 25

### Herstellung von 2-Methyl-3,4-dimethylthio-brombenzol

14,8 g (129 mmol) tert. Butylnitrit und 20 g Kupferpulver werden in 50 ml Dimethyldisulfid vorgelegt und bei 50 bis 55°C eine Lösung von 20g (86 mol) 4-Brom-3-methyl-2-methylthioanilin in 100 ml Dimethyldisulfid zugetropft. Anschließend wird 4 Stunden bei 50°C nachgerührt. Zur Aufarbeitung wird vom Feststoff abgesaugt, mit Methylenchlorid verdünnt und mit verdünnter Salzsäure extrahiert. Die organische Phase wird mit gesättigter wässriger NaHC0₃-Lösung gewaschen, über Natriumsulfat getrocknet, abfiltriert und einrotiert. Überschüssiges Dimethyldisulfid wird am Ölpumpenvakuum entfernt.

Man erhält 19,7 g eines dunklen Öls. Das Produkt kann durch Aufrühren in Methyl-tert.butylether gereinigt werden.

Ausbeute 9,32 g (41 %) Fp.: 70-73°C.

### Beispiel 26

### Herstellung von 2,3-Dimethyl-4-methylthio-brombenzol

603g (5,85 mol) tert. Butylnitrit und 375 g Kupferpulver (5,9 mol) werden in 3000 ml Dimethyldisulfid vorgelegt und bei 50 bis 58°C 761g (3,75 mol) 4-Brom - 2,3-dimethylanilin zugetropft. Anschließend wird 9 Stunden bei 75 bis 80°C nachgerührt. Zur Aufarbeitung wird abgekühlt, vom Rückstand abfiltriert und das Filtrat mit gesättigter wässriger NaHCO₃-Lösung gewaschen. Zur Reinigung des Produkts wird die organische Phase destillativ getrennt. Zunächst wird bei Normaldruck das überschüssige Dimethyldisulfid abgetrennt. Man gewinnt 1870 g Dimethyldisulfid (Reinheit > 97 % nach GC) zurück. Anschließend wird i.Vak. (0,1 mbar) fraktioniert destilliert.

Ausbeute: 523 g (60 %), Reinheit nach GC 99 %.

### Beispiel 27

### (Reaktionsfolge nach Schema 4)

a) Herstellung von 2,3-Dimethylthioanisol
   355 g (3,44 mol) tert. Butylnitrit und 250 g Kupferpulver (3,9 mol) werden in 1250 ml Dimethyldisulfid vorgelegt und bei 50 bis 52°C eine Lösung von 250 g (2,07 mol) 2,3-Dimethylanilin in 1000 ml Dimethyldisulfid zugetropft. Anschließend wird 1,5 Stunden bei 75°C bis 80°C nachgerührt. Zur Aufarbeitung wird abgekühlt, durch Kieselgur abgesaugt und das Filtrat mit gesättigter wässriger NaHCO₃-Lösung gewaschen. Zur Reinigung des Produkts wird die organische Phase destillativ getrennt. Zunächst wird bei Normaldruck das überschüssige Dimethyldisulfid abgetrennt. Man gewinnt 1446 g Dimethyldisulfid (Reinheit >97 % nach GC) zurück. Anschlie-βend wird i.Vak.(0,1 mbar) fraktioniert destilliert.
   Ausbeute: 261,3 g (83 %), Reinheit (nach GC) 97,5 %.
b) Herstellung von 2,3-Dimethyl-4-methylthio-brombenzol
   510 g (3,33 mol) 2,3-Dimethyl-thioanisol werden in 3 l Eisessig vorgelegt und innnerhalb von drei Stunden eine Lösung von 592 g (7,4 mol) Brom in 1 l Eisessig bei Raumtemperatur zugetropft. Die Reaktion ist schwach exotherm. Der Ansatz wird noch 3,5 Stunden bei Raumtemperatur nachgerührt. Anschließend wird der ausfallene Niederschlag abgesaugt, das Filtrat mit 270 g Natriumacetat versetzt und eingeengt. Der Rückstand wird in 2 l Dichlormethan aufgenommen, zweimal mit 21 Natriumhydrogencarbonatlösung und zweimal mit Natriumchloridlösung gewaschen. Die organische Phase wird über Natriumsulfat getrocknet und eingeengt.
   Ausbeute: 615 g (79% ), Reinheit (nach GC) 99,2 %.
c) Herstellung von 2,3-Dimethyl-4-methylsulfonyl-brombenzol
   Zu einer Lösung von 182 g (0,78 mol) 2,3-Dimethyl-4-methylthio-brombenzol und 5,24 g Natriumwolframat-Hydrat in 11 Eisessig werden bei max. 100°C (leichter Rückfluß) 266 g (2,35 mol) 30 %iges Wasserstoffperoxid in 45 Minuten getropft. Der Ansatz wird zwei Stunden bei Raumtemperatur nachgerührt. Zur Aufarbeitung wird auf 7,8 l Eiswasser gegossen und 30 Minuten nachgerührt. Anschließend wird der weiße Rückstand abgesaugt und dreimal mit Wasser gewaschen. Die Kristalle werden über Nacht i.vak. bei 70°C getrocknet.
   Ausbeute: 195 g (94 %), Reinheit (nach GC) 100 %.
d) Herstellung von 3-Brom-2-methyl-6-methylsulfonyl-benzaldoxim
   272,6 g Natriumethylat (3,8 mol) werden in 0,4 l DMF gelöst und bei -20°C bis -15°C eine Lösung von 400 g 2,3-Dimethyl-4-methylsulfonyl-brombenzol (1,52 mol) und 214,6 g (1,977 mol) n-Butylnitrit in 0,8 l DMF zugegeben. Anschlie-βend werden noch einmal 100 g Enatriumethylat zugegeben. Der Ansatz wird insgesamt 5,5 Stunden bei -20°C bis -15°C gerührt.
   Der Ansatz wird auf 41 Eiswasser und 0,4 l Eisessig gegossen und mit insgesamt 41 MtBE extrahiert. Die MtBE-Phase wird mit 11 Natriumhydrogencarbonatlösung und zweimal mit Wasser gewaschen. Die Wasserphasen werden vereinigt. Die MtBE-Phase wird einrotiert und getrocknet. Die Lösung wird eingeengt und der Rückstand an der Ölpumpe getrocknet.
   Ausbeute: 331 g (75 %) gelbbraune Kristalle, Reinheit (nach HPLC) 96,6%.
e) Herstellung von 3-(3-Brom-2-methyl-6-methylsulfonylphenyl)-4,5-dihydroisoxazol
   Zu einer Lösung von 50 g (171 mmol) 3-Brom-2-methyl-6-methylsulfonyl-benzaldoxim in 200 ml Dimethylformamid wird bei 60°C eine kleine Menge N-Chlorsuccinimid gegeben. Nachdem die Reaktion angesprungen ist, werden bei 40-50° insgesamt 23,3 g (171 mmol) N-Chlorsuccinimid zudosiert. Der Ansatz wird noch 30 Minuten nachgerührt bis nach HPLC die Umsetzung vollständig ist. Anschließend wird der Ansatz auf Eiswasser gegossen, der Feststoff abgesaugt, dreimal mit Wasser und zweimal mit n-Pentan gewaschen. Das Hydroxamsäurechlorid wird feucht ohne weitere Reinigung in die nächste Umsetzung eingesetzt. Der Feststoff wird in 250 ml Dichlormethan gelöst und Ethylen durch die Lösung geleitet. Dann werden unter weiterem Durchleiten von Ethylen 20,3 g (200 mmol) Triethylamin zugetropft. Der Ansatz wird ca 72 Stunden bei Raumtemperatur gerührt und dabei noch mehrfach Ethylen eingegast.
   Zur Aufarbeitung wird der Ansatz dreimal mit Wasser gewaschen und das Lösungsmittel abgezogen. Man erhält 49 g bräunliche Kristalle, die nach HPLC 90,6 % Produkt enthalten. Durch Umkristallisieren aus 200 ml i-Propanol kann das Produkt gereinigt werden.
   Ausbeute: 31 g (57%) weiße Kristalle Fp.:133-136°C, Reinheit (nach HPLC) 99,5%.

## Patentansprüche

1. Verfahren zur Herstellung von Verbindungen der Formel XV 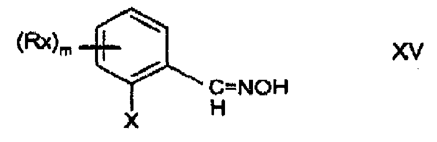 in der die Reste folgende Bedeutung haben:
x NO₂, S(O)ₙRy;
Rx, Ry jeweils ein beliebiger inerter Rest;
m 0, 1, 2, 3 oder 4;
n 0, 1 oder 2;
umfassend die Umsetzung von Verbindungen der Formel XVI 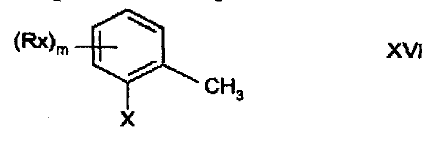 in der die Substituenten die oben angegebene Bedeutung haben, mit einem organischen Nitrit der allgemeinen Formel R-O-NO, wobei R einen aliphatischen oder aromatischen Rest darstellt, unter Einwirkung einer Base, wobei die Umsetzung bei einer Temperatur unterhalb von -20°C in Gegenwart eines dipolar aprotischen Lösungsmittels erfolgt und gegebenenfalls anschließende Umwandlung der Oximgruppe -CH=NOH in Formel XV in die entsprechenden Aldehyde -CHO, Nitrile (-CN) oder Nitriloxide (-CNO).

2. Verfahren nach Anspruch 1, wobei als Lösungsmittel DMF verwendet wird.

3. Verbindungen der Formel XV 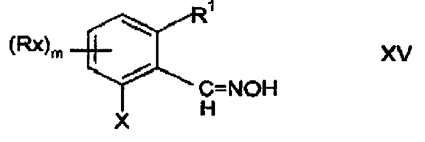 in der die Reste folgende Bedeutung haben:
x SO₂R^{y},
R¹ C₁-C₆-Alkyl
Rx Halogen
Ry C₁-C₆-Alkyl,
m 1,

4. Verfahren nach Anspruch 1 und 2 zur Herstellung von Verbindungen IX 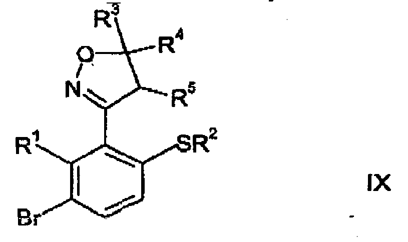 oder X 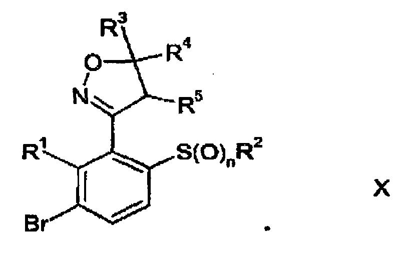 wobei die Substituenten jeweils folgende Bedeutung haben:
R¹ Wasserstoff, C₁-C₆-Alkyl,
R² C₁-C₆-Alkyl,
R³, R⁴, R⁵ Wasserstoff, C₁-C₆-Alkyl oder R⁴ und R⁵ bilden zusammen eine Bindung,
n 1 oder 2.

## Claims

1. A process for preparing compounds of the formula XV 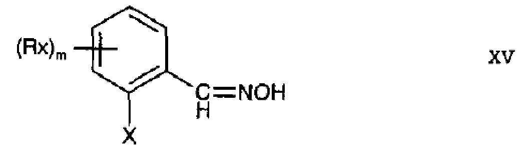 in which the radicals are as defined below:
x is NO₂, S(O)ₙRy;
Rx, Ry are in each case any inert radical;
m is 0, 1, 2, 3 or 4;
n is 0, 1 or 2;
which comprises reacting compounds of the formula XVI 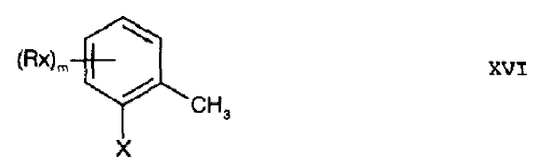 in which the substituents are as defined above with an organic nitrite of the formula R-O-NO, where R is an aliphatic or aromatic radical, in the presence of a base, where the reaction is carried out at a temperature below -20°C in the presence of a dipolar aprotic solvent, optionally followed by conversion of the oxime group -CH=NOH in formula XV into the corresponding aldehydes -CHO, nitriles (- CN) or nitrile oxides (-CNO).

2. The process according to claim 1, where the solvent used is DMF.

3. A compound of the formula XV 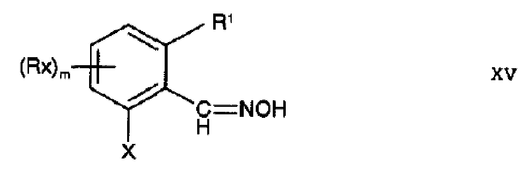 in which the radicals are as defined below:
x is SO₂Ry,
R¹ is C₁-C₆-alkyl,
Rx is halogen,
Ry is C₁-C₆-alkyl,
m is 1.

4. The process according to claims 1 and 2 for preparing compounds IX 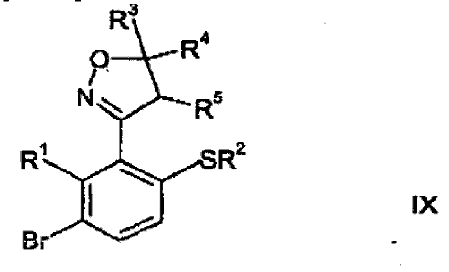 or X 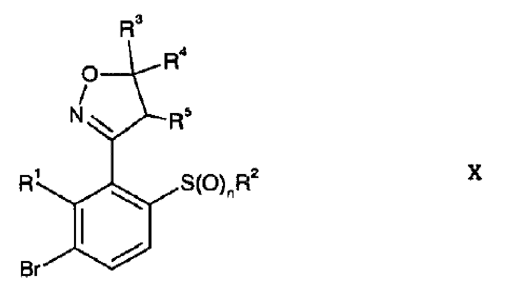 where the substituents are in each case as defined below:
R¹ is hydrogen, C₁-C₆-alkyl,
R² is C₁-C₆-alkyl,
R³, R⁴, R⁵ are hydrogen, C₁-C₆-alkyl, or R⁴ and R⁵ together form a bond,
n is 1 or 2.

## Revendications

1. Procédé de fabrication de composés de formule XV 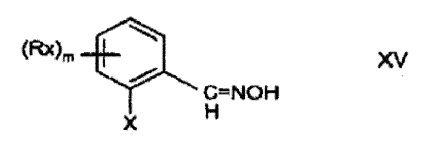 dans laquelle les radicaux ont la signification suivante :
x NO₂, S(O)ₙRy ;
Rx, Ry chacun un radical inerte quelconque ;
m 0, 1, 2, 3 ou 4 ;
n 0, 1 ou 2 ;
comprenant la mise en réaction de composés de formule XVI 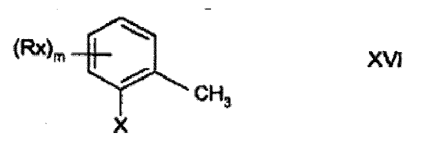 dans laquelle les substituants ont la signification indiquée précédemment, avec un nitrite organique de formule générale R-O-NO, R représentant un radical aliphatique ou aromatique, sous l'effet d'une base, la réaction ayant lieu à une température inférieure à-20 °C en présence d'un solvant aprotique dipolaire, puis éventuellement transformation du groupe oxime-CH=NOH dans la formule XV en les aldéhydes -CHO, les nitriles (-CN) ou les oxydes de nitrile (-CNO) correspondants.

2. Procédé selon la revendication 1, dans lequel le DMF est utilisé en tant que solvant.

3. Composés de formule XV 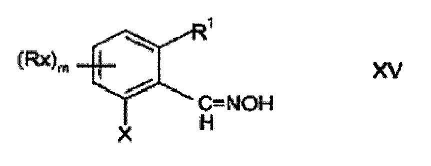 dans laquelle les radicaux ont la signification suivante :
x SO₂R^{y},
R¹ alkyle en C₁-C₆,
Rx halogène,
Ry alkyle en C₁-C₆,
m 1.

4. Procédé selon les revendications 1 et 2, pour la fabrication de composés IX 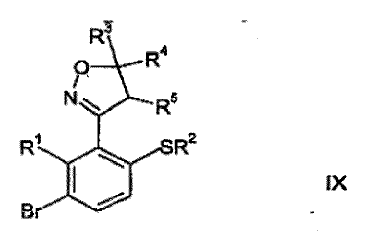 ou X 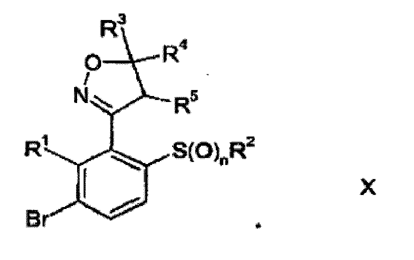 les substituants ayant chacun la signification suivante :
R¹ hydrogène, alkyle en C₁-C₆,
R² alkyle en C₁-C₆,
R³, R⁴, R⁵ hydrogène, alkyle en C₁-C₆, ou R⁴ et R⁵ forment ensemble une liaison,
n 1 ou 2.
